(19) Europäisches Patentamt European Patent Office Office européen des brevets

(11) **EP 4 640 226 A1**

(12) **EUROPEAN PATENT APPLICATION**
published in accordance with Art. 153(4) EPC

(43) Date of publication:
**29.10.2025 Bulletin 2025/44**

(21) Application number: 23910777.4

(22) Date of filing: **27.12.2023**

(51) International Patent Classification (IPC):
*A61K 35/74* (2015.01)    *C12N 1/20* (2006.01)
*A23L 33/135* (2016.01)    *A61P 29/00* (2006.01)
*A61P 35/00* (2006.01)

(52) Cooperative Patent Classification (CPC):
A23L 33/135; A61K 31/7068; A61K 35/74;
A61K 39/395; A61K 45/06; A61P 29/00;
A61P 35/00; A61P 35/02; A61P 37/04; C12N 1/20;
C12R 2001/01

(86) International application number:
**PCT/CN2023/142542**

(87) International publication number:
**WO 2024/140860 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 27.12.2022 CN 202211690225
27.12.2022 CN 202211690259

(71) Applicant: **Moon (Guangzhou) Biotech Co., Ltd.**
**Guangzhou, Guangdong 510535 (CN)**

(72) Inventors:
• **XIAN, Yibo**
**Guangzhou, Guangdong 510535 (CN)**

• **ZHAO, Yingying**
**Guangzhou, Guangdong 510535 (CN)**
• **CHEN, Zhipeng**
**Guangzhou, Guangdong 510535 (CN)**
• **KONG, Ping**
**Guangzhou, Guangdong 510535 (CN)**
• **XIAO, Chen**
**Guangzhou, Guangdong 510535 (CN)**

(74) Representative: **Prüfer & Partner mbB**
**Patentanwälte · Rechtsanwälte**
**Sohnckestraße 12**
**81479 München (DE)**

Remarks:
The complete document including Reference Table(s) and the Sequence Listing(s) can be downloaded from the EPO website

(54) **USE OF CHRISTENSENELLA SP. OR COMPOSITION COMPRISING SAME IN PREVENTION OR TREATMENT OF TUMORS, AND DRUG COMPRISING SAME**

(57)    A composition comprising *Christensenella sp.,* or a metabolite, fermentation broth, or a fermentation broth supernatant thereof, or any combination thereof. The 16S rRNA of *Christensenella sp.* has at least 99%, 99.5%, 99.6%, 99.7%, 99.8%, 99.9%, 99.99%, or 100% identity to the sequence as shown in SEQ ID NO: 1, wherein the *Christensenella sp.* is preferably Gram-negative *Christensenella intestinihominis,* and more preferably the strain with the deposit number GDMCC No: 61117. The composition can prevent or treat tumors or inflammatory diseases, or improve the efficacy of one or more tumor therapies. Experiments prove that in the environment of the intestinal tract of a mouse, *Christensenella sp.* can effectively inhibit the growth speed of various refractory tumors, improve the efficacy of other tumor therapies, can relieve immunosuppression, promote the transcription activity of IFN β, and has potential therapeutic efficacy on various diseases.

FIG. 22A

**Description**

FIELD OF THE INVENTION

The present invention relates to the technical field of microbial drugs, and in particular to the use of a *Christensenella sp.*in the prevention or treatment of tumor.

BACKGROUND OF THE INVENTION

Currently, cancer treatments are still focused on surgery, chemotherapy, radiotherapy and immunotherapy. Due to the microenvironment of the tumor itself, such as hypoxia and disorganized microvascular, the efficacy of various treatments is limited, while live bacterial drugs have the ability to change the microenvironment of the tumor and activate the anti-tumor immune, and therefore it is expected to become a new strategy for cancer treatment. In recent years, an increasing number of studies have demonstrated that the microbiota can influence cancer treatment outcomes by modulating the tumor microenvironment or the systemic immune system. Clinical studies indicate that microbiome-targeted interventions, such as FMT, can reverse tolerance to immune checkpoint drugs in melanoma patients, improve the therapeutic efficacy of immune checkpoint drugs, and improve the prognosis and adverse effects of patients in the early stages of treatment. As a result, microbiome-targeted interventions have emerged as a novel frontier of cancer drug development, and the pipeline of live bacterial drugs targeting cancer has become one of the most popular indications for live bacterial drug development, second only to *Clostridioides difficile* infection.

Dysbiosis of intestinal microbiota can increase the incidence of colorectal cancer, while the metabolites of some intestinal microbes are able to directly slow down carcinogenesis or inhibit tumorigenesis. Not only intestine-related colon cancer and rectal cancer, but also the breast cancer, liver cancer and the like can be affected by the intestinal microbiota. There is growing evidence indicating that intestinal microbes can influence tumor formation, tumor development, and effectively cooperate with tumor treatments. Therefore, how to regulate the immune and inflammatory responses induced by intestinal microbiota, so that they do not induce oncogenesis but also enhance the efficacy of antitumor drugs, is still under continuous exploration and investigation. Addressing these issues could have significant implications for innovations and changes in the field of tumor therapy in the future.

Prior art, such as the probiotic field or FMT microbiota transplantation, usually employs a mixture of multiple microbes to act in the intestine. Some prior art also utilizes a mixture of multiple bacteria as an antitumor drug. However, the mechanism of multiple bacteria action on indications is more complex, and the influence of interactions between different bacteria has not been well studied. As a live bacterial drug, the use of multiple bacteria can disrupt the homeostasis of the intestinal microbiota, in contrast to single bacteria, which have a lesser impact on the homeostasis of the intestinal microbiota. Moreover, the use of bacteria mixture requires additional consideration of whether the individual bacteria might affect each other's activity. It is also not clear whether it is the synergistic effect of the bacteria mixture that makes it play a role in the immune process or a single type of bacteria that plays a role in the immune process.

*Christensenella* is a genus of intestinal commensal bacteria that has been identified in recent years, and comprises an extremely limited number of species, with only six species described to date, all of which have been reported in recent years. Research on the *Christensenella* genus is also very limited; moreover, there are very few patents and literatures related to tumors.

Therefore, it has become necessary and urgent to explore and identify an anaerobic microorganism that can be used to inhibit tumor growth for application in the prevention or treatment of tumors or to enhance the efficacy of tumor therapeutic agents or cellular therapies.

In view of this, the present invention is proposed.

SUMMARY OF THE INVENTION

It is an object of the present invention to provide a *Christensenella sp.,* in particular *Christensenella intestinihominis,* and more particularly *Christensenella sp.* MNH04863, for use in the prevention or treatment of disease, in particular in the prevention or treatment of solid tumor.

Specifically, in a first aspect of the present invention, provided is a microbial agent comprising a *Christensenella* strain, or a metabolite, a fermentation culture, or a fermentation culture supernatant thereof, or any combination thereof.

In some embodiments, the microbial agent can be used for preventing or treating a tumor or improving the tumor treatment efficacy; or preventing or treating an inflammatory disease.

In some embodiments, the microbial agent comprises an effective amount of a *Christensenella* strain, or a metabolite, a fermentation culture, or a fermentation culture supernatant thereof, or any combination thereof.

In some embodiments, the microbial agent further comprises one or more pharmaceutically acceptable carriers or excipients or adjuvants.

In some embodiments, the Christensenella sp. comprises one or more bacteria selected from the following: Christensenella intestinihominis, Christensenella massiliensis, Christensenella minuta, Christensenella timonensis or any combination thereof. In some embodiments, the Christensenella sp. comprises at least one strain of Christensenella intestinihominis. In some embodiments, the Christensenella sp. comprises Christensenella intestinihominis in combination with at least one other Christensenella sp. selected from Christensenella massiliensis, Christensenella minuta, or Christensenella timonensis. In some embodiments, the Christensenella sp. comprises Christensenella intestinihominis and Christensenella minuta.

In some embodiments, the *Christensenella sp.* is Gram-negative, in particular a Gram-negative *Christensenella intestinihominis.*

In some embodiments, a metabolite of the *Christensenella sp.* includes a short chain fatty acid, the short chain fatty acid comprising at least one of acetic acid, propionic acid, butyric acid, valbutyric acid, valeric acid, and hexanoic acid; preferably, the *Christensenella sp.* is capable of high-yield production of butyric acid and acetic acid; wherein the yield of butyric acid is not less than 200 $\mu$g/g and/or the yield of acetic acid is not less than 500 $\mu$g/g; more preferably, the yield of butyric acid yield is not less than 360 $\mu$g/g and/or the yield of acetic acid is not less than 2694 $\mu$g/g.

In some embodiments, the *Christensenella sp.* comprises a strain with a deposit number of GDMCC NO: 61117.

In some embodiments, the 16S rRNA sequence of the *Christensenella sp.* is as shown in SEQ ID NO: 1.

In some embodiments, the microbial agent is a fermentation culture or a fermentation culture supernatant of the *Christensenella sp..* Preferably, the microbial agent is a fermentation culture or a fermentation culture supernatant of the strain with the deposit number of GDMCC No: 61117.

In some embodiments of the present invention, the fermentation culture or fermentation culture supernatant is a fermentation culture or fermentation culture supernatant obtained by culturing under anaerobic culture conditions using a liquid medium; preferably the liquid medium is MM01 liquid medium; preferably the culture conditions comprise: an culture temperature of 30-42°C, for example 30°C, 31°C, 32°C, 33°C, 34°C, 35°C, 36°C, 37°C, 38°C, 39°C, 40°C, 41°C, 42°C or any value therebetween; and/or the pH of the medium is 6-10, preferably pH 7-9, for example 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9 or any value therebetween.

In some embodiments, the tumor is selected from solid tumors, soft tissue tumors, hematopoietic tumors, glandular tumors, or metastatic tumors; or the tumor is a virus-induced tumor or a bacteria-induced tumor. In some embodiments, the tumor is selected from at least one of the following: liver cancer, colon cancer, rectal cancer, colorectal cancer, lung cancer, breast cancer, cervical cancer, ovarian cancer, pancreatic cancer, cholangiocarcinoma, kidney cancer, glioma, liposarcoma, chondrosarcoma, fibrosarcoma, melanoma, cervical cancer, neuroblastoma, lymphoma, leukemia, gastric cancer, hematological malignancies, prostate cancer, small cell lung cancer, squamous cell carcinoma, head and neck neoplasm, bladder cancer, nasopharyngeal carcinoma, multiple myeloma, tumor induced by human papillomavirus (HPV) infection, tumor induced by hepatitis B virus (HBV) infection, tumor induced by hepatitis C virus (HCV) infection, tumor induced by human immunodeficiency virus (HIV) infection, tumor induced by *Helicobacterpylori* infection, and tumor induced by EBV infection, or tumor induced by *Fusobacterium nucleatum* infection.

In some embodiments, the inflammatory disease is selected from the following: rheumatic disease, connective tissue disease, systemic vasculitis, dermatological inflammatory disease, sepsis/systemic inflammatory response syndrome, acute respiratory distress syndrome, gastritis, pneumonia, or hepatitis, and preferably the inflammatory disease is selected from rheumatoid arthritis, systemic sclerosis, systemic lupus erythematosus, sicca syndrome, Reiter's syndrome, systemic juvenile idiopathic arthritis, spondyloarthropathies, giant cell arteritis, polymyalgia rheumatica, aorto-arteritis, polyarteritis nodosa, Kawasaki disease, ANCA-associated vasculitis, immune-complex-mediated vasculitis, Behcet's syndrome, relapsing polychondritis, sarcoidosis, psoriasis, psoriatic arthritis, eczema, chronic urticaria, neutrophilic dermatosis, acute or chronic gastritis, alopecia areata, asthma, bronchitis, or alcoholic or autoimmune hepatitis.

In some embodiments, the microbial agent comprises *Christensenella sp.* with a viable count of $1\times10^9 \sim 3\times10^{10}$ CFU/mL or $1\times10^9 \sim 3\times10^{10}$ CFU/g.

In a second aspect of the present invention, provided is a composition comprising an effective amount of (1) a microbicidal agent according to the first aspect of the present invention, and (2) at least one other agent, the at least one other agent is selected from a chemotherapeutic drug, a radiotherapeutic drug, a targeted drug, a photosensitizer, a photothermal agent, an immunotherapeutic agent, a drug for the treatment of diabetes mellitus or any combination thereof.

In some embodiments, the chemotherapeutic drug is selected from (a) alkylating agents, such as nitrogen mustards (e.g., nitrogen mustard, cyclophosphamide, ifosfamide, melphalan, chlorambucil), ethylenimines and methylmelamines (e.g., hexamethylmelamine, thiotepa), alkylsulfonates (e.g., busulfan), nitrosoureas (e.g., carmustine, lomustine, chlorozotocin, streptozotocin), and triazines (e.g., dacarbazine); (b) anti metabolites such as folate analogs (e.g., methotrexate), pyrimidine analogs (e.g., gemcitabine, capecitabine, 5-fluorouracil, fluorouracil, cytarabine, azauracil), and purine analogs and related substances (e.g., 6-mercaptopurine, 6-thioguanine, pentostatin); (c) natural products such as the vinca alkaloids (e.g., vinblastine, vincristine), epipodophyllotoxins (e.g., etoposide, teniposide), antibiotics (e.g., actinomycin D, daunorubicin, doxorubicin, mitomycin, epirubicin, bleomycin, plicamycin, and mitoxantrone), enzymes (e.g., L-asparaginase), and biological response modifiers (e.g., interferon-a); (d) other agents such as platinum coordination complexes (e.g., cisplatin, carboplatin), substituted ureas (e.g., hydroxyurea), methylhydrazine derivatives (e.g., procarbazine) and adrenocortical suppressants (e.g., paclitaxel and mitotane). In some embodiments, the chemotherapeutic drug comprises at least one selected from gemcitabine, oxaliplatin, paclitaxel, camptothecin, 5-fluorouracil, cisplatin, doxorubicin, mitomycin, or epirubicin.

In some embodiments, the radiotherapeutic drug comprises a drug used in external radiation therapy, internal radiation therapy, radioimmunotherapy, or intraoperative radiation therapy (IORT).

In some embodiments, the photosensitizer comprises at least one selected from boron dipyrromethene, chlorin or rose bengal.

In some embodiments, the photothermal agent comprises at least one selected from indocyanine green, new indocyanine green, or gold nanorods.

In some embodiments, the immunotherapeutic agent comprises an immune checkpoint inhibitor, a co-stimulatory molecule inhibitor, a dendritic cell, a CAR-T cell, a cytokine, or an adoptive T cell. In some embodiments, the immune checkpoint inhibitor is selected from inhibitors capable of binding, blocking, and/or inhibiting the activity of one or more of CTLA-4, PDL1, PDL2, PD1, BTLA, HVEM, TIM3, GAL9, LAG3, VISTA, KIR, 2B4, CD160, and CGEN-15049. In some embodiments, the immune checkpoint inhibitor comprises at least one selected from an anti-PD-1 antibody, an anti-CTLA-4 antibody, an anti-PD-L1 antibody, or a PD-L1 inhibitor. In some embodiments, the immune checkpoint inhibitor is selected from durvalumab, atezolizumab, avelumab, pembrolizumab, nivolumab, ipilimumab, or any combination thereof. In some embodiments, a dosage of the anti-PD-1 antibody is 5~15 mg/kg, such as 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, or any value therebetween.

In some embodiments, the composition further comprises at least one probiotic. In some embodiments, the at least one probiotic is selected from: *Bifidobacterium spp., Lactobacillus spp., Lactococcus spp., Lacticaseibacillus spp., Pediococcus acidilactici, Pediococcus pentosaceus,* and/or *Saccharomyces boulardii.*

In some embodiments, the probiotic is selected from: Bifidobacterium adolescentis, Bifidobacterium animalis subsp. animalis, Bifidobacterium animalis subsp. lactis, Bifidobacterium bifidum, Bifidobacterium breve, Bifidobacterium longum subsp. longum, Bifidobacterium longum subsp. infantis, Lactobacillus acidophilus, Lactobacillus crispatus, Lactobacillus delbrueckii subsp. Bulgaricus, Lactobacillus delbrueckii subsp. Lactis, Lactobacillus gasseri, Lactobacillus helveticus, Lactobacillus johnsonii, Lactobacillus kefiranofaciens subsp. Kefiranofaciens, Lacticaseibacillus casei, Lacticaseibacillus paracasei, Lacticaseibacillus rhamnosus, Limosilactobacillus fermentum, Limosilactobacillus reuteri, Lactiplantibacillus plantarum, Ligilactobacillus salivarius, Latilactobacillus curvatus, Latilactobacillus sakei, Streptococcus salivarius subsp. thermophilus, Lactococcus lactis subsp. lactis, Lactococcus lactis subsp. lactis biovar diacetylactis, Lactococcus cremoris, Propionibacterium freudenreichii subsp. shermanii, Acidipropionibacterium acidipropionici, Leuconostoc mesenteroides subsp. mesenteroides, Pediococcus acidilactici, Pediococcus pentosaceus, Weizmannia coagulans, Mammaliicoccus vitulinus, Staphylococcus xylosus, Staphylococcus carnosus, and Kluyveromyces marxianus.

In some embodiments, the composition is in the form of a liquid, foam, cream, spray, powder (e.g., lyophilized powder), or gel. In some embodiments, the composition comprises one or more selected from following: a buffering agent (e.g., sodium bicarbonate), infant formula milk or sterile human milk or other reagents that allow for bacterial survival and growth (for example, survival in the acidic environment of the stomach and growth in the intestinal environment), a lyoprotectant, a preservative, a stabilizing agent, a binder, a compacting agent, a lubricant, a dispersion enhancer, a disintegrating agent, an antioxidant, a flavoring agent, a sweetener, and a coloring agent. In some embodiments, the composition may be formulated as a frozen composition, such as snap-frozen, dried, or lyophilized for storage and/or transportation. Furthermore, the composition may be administered alone or in combination with a carrier such as a pharmaceutically acceptable carrier or a biocompatible scaffold.

In some embodiments, the composition is for preventing or treating a tumor as described in the first aspect of the present invention. In some embodiments, the composition is for improving the efficacy of one or more tumor therapies. In some embodiments, the composition is for preventing or treating an inflammatory disease as described in the first aspect of the present invention.

In some embodiments, the tumor is selected from solid tumors, soft tissue tumors, hematopoietic tumors, glandular tumors, or metastatic tumors; or the tumor is a virus-induced tumor or a bacteria-induced tumor. In some embodiments, the tumor is selected from at least one of the following: liver cancer, colon cancer, rectal cancer, colorectal cancer, lung cancer, breast cancer, cervical cancer, ovarian cancer, pancreatic cancer, cholangiocarcinoma, kidney cancer, glioma, liposarcoma, chondrosarcoma, fibrosarcoma, melanoma, cervical cancer, neuroblastoma, lymphoma, leukemia, gastric cancer, hematological malignantcies, prostate cancer, small cell lung cancer, squamous cell carcinoma, head and neck neoplasm, bladder cancer, nasopharyngeal carcinoma, multiple myeloma, tumor induced by human papillomavirus (HPV) infection, tumor induced by hepatitis B virus (HBV) infection, tumor induced by hepatitis C virus (HCV) infection, tumor induced by human immunodeficiency virus (HIV) infection, tumor induced by Helicobacterpylori infection, and tumor induced by EBV infection, or tumor induced by *Fusobacterium nucleatum* infection.

In some embodiments, the composition has one or more features selected from the following (1) to (7):

(1) the composition is in an infant-applicable dosage form, a child-applicable dosage form, or an adult-applicable dosage form;

(2) the composition is in a dosage form for gastrointestinal administration or a dosage form for parenteral administration;

(3) the composition is an oral preparation or injectable preparation;

(4) the strain is at least partially capable of proliferating in the intestinal tract of a subject;

(5) the strain or metabolite, culture or culture supernatant thereof presents in the pharmaceutical composition in a mass percentage of 1%-80%; for example, 2%-70%, 5%-60%, 10%-50%, or 20%-40%, specifically, for example, 5%, 10%, 15%, 20%, 25%, 30%, 35%, 40%, 45%, 50%, 55%, 60%, 65%, 70%, 75%, 80% or any value therebetween, preferably 45%, 50%, 55% or 60%;

(6) the strain is in a form selected from: live bacteria, attenuated bacteria, killed bacteria, lyophilized bacteria or irradiated bacteria; and

(7) each gram of the composition comprises $1\times10^3$ to $1\times10^{13}$ colony forming units (CFU) of the strain; e.g., $1\times10^4$ to $1\times10^{12}$, $1\times10^5$ to $1\times10^{11}$, or $1\times10^6$ to $1\times10^{10}$ colony forming units (CFU) of the strain, specifically, e.g., $1\times10^3$, $5\times10^3$, $1\times10^4$, $5\times10^4$, $1\times10^5$, $5\times10^5$, $1\times10^6$, $5\times10^6$, $1\times10^7$, $5\times10^7$, $1\times10^8$, $5\times10^8$, $1\times10^9$, $5\times10^9$, $1\times10^{10}$, $5\times10^{10}$, $1\times10^{11}$, $5\times10^{11}$, $1\times10^{12}$, $5\times10^{12}$, $1\times10^{13}$, $5\times10^{13}$ or any value therebetween of the colony-forming units (CFU) of the strain.

In some embodiments, the bacterial strain in the composition is freeze-dried or spray-dried. In some embodiments, the bacterial strain in the composition is spray-dried. In some embodiments, the bacterial strain in the composition is electrostatic spray-dried. In some embodiments, the bacterial strain in the composition is freeze-dried or spray-dried and wherein it is viable. In some embodiments, the bacterial strain in the composition is freeze-dried or spray-dried and wherein it is capable of partially or fully colonizing the intestine. In some embodiments, the freeze-dried bacterial strain is subjected to reconstitution prior to administration. In some embodiments, the reconstitution is performed using a diluent as described herein.

In some embodiments, the composition of the present application is administered orally. In some embodiments, the composition is an enteric formulation. In some embodiments, the enteric formulation comprises an enteric coating. In some embodiments, the composition is in an enteric-coated dosage form. For example, the enteric formulation may be an enteric-coated granule, enteric-coated tablet or enteric-coated capsule and the like. In some embodiments, the composition is a soft capsule formulation. In some embodiments, the composition is a capsule formulation. In some embodiments, the capsule formulation may be a hard capsule or a soft capsule; or the capsule formulation may be a sustained-release capsule, a controlled-release capsule, or an enteric-coated capsule, or the alternatively, the capsule formulation may be a microencapsulated capsule, or a microcapsule, etc.

In some embodiments, the composition is a pharmaceutical composition. In some embodiments, the composition is a health care product or a food product.

In a third aspect of the present invention, provided is a pharmaceutical package or kit comprising a individually packaged or placed component (1) and component (2) of a composition according to the second aspect of the present invention, and optionally a product instruction.

In some embodiments, the individual component is provided in a concentrated amounts in the pharmaceutical packet or kit.

In some embodiments, the component (1) and component (2) further independently comprise one or more pharmaceutically acceptable adjuvants.

In some embodiments, the pharmaceutical package or kit is capable of being used for preventing or treating a tumor as described in the first aspect of the present invention or improving tumor treatment efficacy; or preventing or treating an inflammatory disease as described in the first aspect of the present invention.

In a fourth aspect of the present invention, provided is a use of a microbicidal agent as described in the first aspect of the present invention or a composition as described in the second aspect of the present invention in the preparation of a medicament for preventing or treating a tumor or an inflammatory disease.

In some embodiments, the tumor is selected from a solid tumor, a soft tissue tumor, a hematopoietic tumor, a glandular tumor, or a metastatic tumor.

In some embodiments, the tumor is selected from one or more of liver cancer, colon cancer, rectal cancer, colorectal cancer, lung cancer, breast cancer, cervical cancer, ovarian cancer, pancreatic cancer, cholangiocarcinoma, kidney cancer, glioma, liposarcoma, chondrosarcoma, fibrosarcoma, melanoma, cervical cancer, neuroblastoma, lymphoma, leukemia, gastric cancer, hematologic malignancies, prostate cancer, small cell lung cancer, squamous cell carcinoma, head and neck neoplasm, bladder cancer, nasopharyngeal carcinoma or multiple myeloma.

In some embodiments, the tumor is selected from a tumor induced by a virus or a tumor induced by a bacterium, such as a tumor induced by human papillomavirus (HPV) infection, hepatitis B virus (HBV) infection, hepatitis C virus (HCV) infection, human immunodeficiency virus (HIV) infection, *Helicobacter pylori* (*H. pylor*) infection, EB virus infection, or *Fusobacterium nucleatum* infection.

In some embodiments, the inflammatory disease is selected from the following: rheumatic disease, connective tissue disease, systemic vasculitis, dermatological inflammatory disease, sepsis/systemic inflammatory response syndrome, acute respiratory distress syndrome, gastritis, pneumonia, or hepatitis, and preferably the inflammatory disease is selected from rheumatoid arthritis, systemic sclerosis, systemic lupus erythematosus, sicca syndrome, Reiter's syndrome, systemic juvenile idiopathic arthritis, spondyloarthropathies, giant cell arteritis, polymyalgia rheumatica, aorto-arteritis, polyarteritis nodosa, Kawasaki disease, ANCA-associated vasculitis, immune-complex-mediated vasculitis, Behcet's syndrome, relapsing polychondritis, sarcoidosis, psoriasis, psoriatic arthritis, eczema, chronic urticaria, neutrophilic dermatosis, acute or chronic gastritis, alopecia areata, asthma, bronchitis, or alcoholic or autoimmune hepatitis.

In some embodiments, the treating a tumor comprises improving the efficacy of one or more tumor therapies.

In some embodiments, the medicament exerts antitumor effects through at least one of the following matterns: (a) inhibiting tumor volume growth; (b) inhibiting tumor weight gain; (c) inhibiting tumor cell proliferation; (d) improving tumor treatment response rate; (e) improving the therapeutic efficacy of an immunosuppression drug, such as improving the therapeutic efficacy of a PD-1 drug; (f) reducing the resistance of an immunotherapeutic agent, e.g., an anti-PD-1 antibody resistance; (g) preventing or inhibiting the spread or metastasis of tumor cells; (h) relieving immunosuppression; (i) inhibiting a tumor via at least one of short-chain fatty acids or short-chain fatty acid salts including acetic acid or acetate, propionic acid or propionate, butyric acid or butyrate, and valeric acid or valerate in SCFAs; (j) inhibiting a tumor by modulating the immune system in a subject to exert an immunomodulatory effect; (k) promoting senescence or apoptosis of cancer cells or tumor cells; (1) stimulating the immune system in a subject; and (m) inhibiting angiogenesis within tumor tissue.

In some embodiments, the medicament achieves treatment or prevention of a tumor by activating the STING signaling pathway, promoting IFNβ transcriptional activity, and/or inhibiting the proportion of MDSC in the tumor microenvironment.

In some embodiments, the medicament performs immunomodulation on macrophages and/or primary PBMC cells and/or monocyte-derived dendritic cells, and thereby inhibiting the growth of the tumor. In some embodiments, the *Christensenella sp.* is capable of increasing the production of one or more pro-inflammatory cytokines and/or chemokines by human macrophages, monocytes, peripheral blood monocytes, and/or monocyte-derived dendritic cells, and/or is capable of increasing the infiltration of T cells in a tumor. In some embodiments, the pro-inflammatory cytokine is selected from one or more of interferon-y, interleukin IL-1β, IL-6, IL-12, IL-23P40, tumor necrosis factor TNFα; and/or, the chemokine is selected from one or more of MCP-1 (CCL2), MIG (CXCL9), RANTES (CCL5), IP-10 (CXCL-10), or IL-8. In some embodiments, the immunomodulation of macrophages by the medicament comprises that the *Christensenella sp.* induces differentiation of M10-type macrophages to M1-type macrophages; wherein the levels of the pro-inflammatory factors TNFα and IL-1β are significantly upregulated in the macrophages, and/or the levels of the chemokines MCP-1 and IL-8 are significantly upregulated in the macrophages. In some embodiments, the immunomodulation of primary PBMCs by the medicament comprises that the *Christensenella sp.* induces a significant increase of the chemokine IL-8 in primary PBMC cells; and/or the *Christensenella sp.* induces an expression increase of the inflammatory factors IL-1β, IL-12/IL-23P40, TNFα, IL-6, or IFNγ in primary PBMC cells.

In a fifth aspect of the present invention, provided is use of a microbial agent as described in the first aspect of the present invention or a composition as described in the second aspect of the present invention in the preparation of a medicament, wherein the medicament is used as an immunomodulatory agent, such as an immunomodulatory agent for relieving immunosuppression; in particular, the medicament is used as an immunomodulatory agent for myeloid-derived suppressor cells (MDSCs) in a tumor patient.

In some embodiments, the type of tumor is one that leads to an elevated MDSC proportion, such as a cold tumor (a cold tumor is an immune cell-suppressive tumor characterized by low infiltration of T cells within and around the tumor); a cold tumor is a tumor characterized by low infiltration of T cells within and around the tumor, resulting in a limited therapeutic effect of an anti-PD-1 antibody or an anti-PD-L1 antibody. The medicament prepared from *Christensenella sp.* of the present invention can relieve immunosuppression, reduce MDSC proportions in the tumor microenvironment, and effectively inhibit the immune cell-suppressive tumor, thereby achieving antitumor effects.

In some embodiments, the tumor is selected from a drug-resistant tumor, preferably a tumor resistant to an immunotherapeutic agent. In some embodiments, the medicament may be used for a tumor resistant to anti-PD-1 antibody, anti-CTLA-4 antibody, anti-PD-L1 antibody, and/or PD-L1 inhibitor, and in particular for a tumor that is clinically resistant to anti-PD-1 antibody or anti-PD-L1 antibody.

In some embodiments, the *Christensenella sp.* inhibits tumor growth by suppressing the proportion of MDSC in the tumor microenvironment.

In some embodiments, the tumor comprises one or more of melanoma, glioma, head and neck neoplasm, lung cancer, colorectal cancer, bladder cancer, nasopharyngeal carcinoma, liver cancer, kidney cancer, pancreatic cancer, or lymphoma.

In a sixth aspect of the present invention, provided is use of a microbial agent as described in the first aspect of the present invention or a composition as described in the second aspect of the present invention in the preparation of a medicament, wherein the medicament is used to improve the efficacy of a tumor treatment.

In some embodiments, the tumor treatment comprises the use of one or more chemotherapeutic drugs, radiotherapeutic agents, targeted agents, chemotherapeutic photosensitizers, photothermal agents, and/or immunotherapies.

In some embodiments, the one or more chemotherapeutic drugs is selected from (a) alkylating agents, such as nitrogen mustards (e.g., nitrogen mustard, cyclophosphamide, ifosfamide, melphalan, chlorambucil), ethylenimines and methylmelamines (e.g., hexamethylmelamine, thiotepa), alkylsulfonates (e.g., busulfan), nitrosoureas (e.g., carmustine, lomustine, chlorozotocin, streptozotocin), and triazines (e.g., dacarbazine); (b) anti metabolites such as folate analogs (e.g., methotrexate), pyrimidine analogs (e.g., gemcitabine, capecitabine, 5-fluorouracil, fluorouracil, cytarabine, azauracil), and purine analogs and related substances (e.g., 6-mercaptopurine, 6-thioguanine, pentostatin); (c) natural products such as the vinca alkaloids (e.g., vinblastine, vincristine), epipotophyllotoxins (e.g., etoposide, teniposide), antibiotics (e.g., actinomycin D, daunorubicin, doxorubicin, mitomycin, epirubicin, bleomycin, plicamycin, and mitoxantrone), enzymes (e.g., L-asparaginase), and biological response modifiers (e.g., interferon-a); (d) other agents such as platinum coordination complexes (e.g., cisplatin, carboplatin), substituted ureas (e.g., hydroxyurea), methylhydrazine derivatives (e.g., procarbazine) and adrenocortical suppressants (e.g., paclitaxel and mitotane). In some embodiments, the chemotherapeutic drug includes at least one selected from gemcitabine, oxaliplatin, paclitaxel, camptothecin, 5-fluorouracil, cisplatin, doxorubicin, mitomycin, or epirubicin.

In some embodiments, the one or more radiotherapies include external radiation therapy, internal radiation therapy, radioimmunotherapy, or intraoperative radiation therapy (IORT).

In some embodiments, the photosensitizer comprises at least one selected from boron dipyrromethene, chlorin or rose bengal.

In some embodiments, the photothermal agent comprises at least one selected from indocyanine green, new indocyanine green, or gold nanorods.

In some embodiments, the one or more immunotherapies include an immune checkpoint inhibitor, a co-stimulatory molecule inhibitor, a dendritic cell therapy, a CAR-T cell therapy, a cytokine therapy, or an adoptive T cell therapy. In some embodiments, the immune checkpoint inhibitor is selected from inhibitors capable of binding, blocking, and/or inhibiting the activity of one or more of CTLA-4, PDL1, PDL2, PD1, BTLA, HVEM, TIM3, GAL9, LAG3, VISTA, KIR, 2B4, CD160, and CGEN-15049. In some embodiments, the immune checkpoint inhibitor comprises at least one selected from an anti-PD-1 antibody, an anti-CTLA-4 antibody, an anti-PD-L1 antibody, or a PD-L1 inhibitor. In some embodiments, the immune checkpoint inhibitor is selected from durvalumab, atezolizumab, avelumab, pembrolizumab, nivolumab, ipilimumab, or any combination thereof.

In some embodiments, the Christensenella sp. comprises one or more bacteria selected from the following: Christensenella intestinihominis, Christensenella massiliensis, Christensenella minuta, Christensenella timonensis or any combination thereof. In some embodiments, the Christensenella sp. comprises at least one strain of Christensenella intestinihominis. In some embodiments, the Christensenella sp. comprises Christensenella intestinihominis in combination with at least one other Christensenella sp. selected from Christensenella massiliensis, Christensenella minuta, or Christensenella timonensis. In some embodiments, the Christensenella sp. comprises Christensenella intestinihominis and Christensenella minuta.

In some embodiments, a metabolite of the *Christensenella sp.* includes a short chain fatty acid, wherein the short chain fatty acid comprises at least one of acetic acid, propionic acid, butyric acid, valbutyric acid, valeric acid, and hexanoic acid; preferably, the *Christensenella sp.* is capable of high-yield production of butyric acid and acetic acid; wherein the yield of butyric acid is not less than 200 $\mu$g/g and/or the yield of acetic acid is not less than 500 $\mu$g/g; more preferably, the yield of butyric acid yield is not less than 360 $\mu$g/g and/or the yield of acetic acid is not less than 2694 $\mu$g/g.

In some embodiments, the *Christensenella sp.* comprises a strain with a deposit number of GDMCC NO: 61117.

In some embodiments, the medicament comprises an effective amount of a *Christensenella sp.,* or a metabolite or culture supernatant thereof, or any combination thereof.

In a seventh aspect of the present invention, provided is a method for preventing or treating a tumor, the method including administering to a subject in need a microbial agent as described in the first aspect of the present invention or a composition as described in the second aspect of the present invention.

In some embodiments, the method further includes a step of screening a candidate to receive the microbial agent or composition for treatment, which includes:

- collecting blood and/or tumor tissue samples from the subject; and
- determining the level of MDSC in the blood and/or tumor tissue samples.

Those subjects who have a high frequency or absolute number of MDSC in the blood or who are positive in terms of MDSC infiltration in the tumor tissue are considered candidates for receiving the treatment of the present invention.

In some embodiments, the candidate is a subject that is resistant to an immunotherapeutic agent, particularly a subject that is resistant to an anti-PD-1 antibody, an anti-CTLA-4 antibody, an anti-PD-L1 antibody, and/or a PD-L1 inhibitor.

Summary

Beneficial effects of the present invention:
The present invention has been developed by finding that *Christensenella sp.* (in particular, *Christensenella sp.* MNH04863) has at least one of a butyric acid-producing pathway or a 4-aminobutyric acid-producing pathway and is capable of producing various short-chain fatty acids, in particular butyric acid, in high yields, which is capable of inhibiting the activity of histone acetylase (e.g., class I, class II, class III and/or class IV histone deacetylases), enhancing the anti-tumor activity of cytotoxic T cells. In addition, the present invention has found that *Christensenella sp.* or a metabolite or culture supernatant thereof can effectively inhibit the growth rate of lung tumor in lung cancer/liver cancer/pancreatic cancer homologous tumor model mice, as well as modulate the immune activity of macrophages and/or primary PBMC cells and/or monocyte-derived dendritic cells, resulting in the determination that *Christensenella sp.* MNH04863 has anti-tumor potential.

The applicant has further confirmed via experiments that *Christensenella sp.* as described herein either alone or in combination with anti-PD-1 antibody and gemcitabine have both preventive and therapeutic effects on lung tumor, liver tumor, and pancreatic tumor.

Accordingly, the present invention provides a wide range of use of *Christensenella sp.* in the prevention or treatment of various tumors, including, but not limited to, inhibiting the proportion of MDSC in the tumor microenvironment, promoting the transcription of the type I interferon IFNβ, activating the STING signaling pathway, enhancing the anti-tumor activity of cytotoxic T cells, enhancing an immune response in a subject, inhibiting the growth of tumor cells, inhibiting the spread of tumor cells, inhibiting immune escape of tumor, enhancing the efficacy of therapeutic agent, increasing the tumor response rate, regulating the level of one or more commensal microorganisms in the intestine in a subject, upregulating pro-inflammatory cytokines and chemokines, decreasing intestinal barrier permeability and/or upregulating protective immunogenic cytokines, modulating chemokines, and increasing T-cell infiltration in a tumor and the like. It is particularly suitable for treating refractory and drug-resistant cancer.

The present invention also provides the use of *Christensenella sp.,* or a metabolite, a fermentation culture, or a fermentation culture supernatant thereof as an immunomodulator, in particular for relieving immunosuppression to achieve antitumor uses; in particular, the immunomodulator is used as an immunomodulator of myeloid-derived suppressor cells (MDSC) in tumor patient. That is, the *Christensenella sp.,* or the metabolite, the fermentation culture, or the fermentation culture supernatant thereof according to the present invention can be used for the treatment or prevention of immune cell-suppressive tumor: the type of the tumor can be a tumor that increases the proportion of MDSCs, such as a cold tumor. The cold tumor is a type of immune cell-suppressive tumor characterized by a tumor type with low T-cell infiltration within and around the tumor; the cold tumor is a class of tumors that result in limited clinical efficacy of anti-PD-1 antibody or anti-PD-L1 antibody. The medicament prepared from *Christensenella sp.* provided by the present invention can inhibit the proportion of MDSC in the tumor microenvironment by relieving immunosuppression, enabling immune cell-suppressive tumor to be effectively suppressed and achieving antitumor use.

Such as a cold tumor (a cold tumor is an immune cell-suppressive tumor characterized by low infiltration of T cells within and around the tumor); a cold tumor is a tumor characterized by low infiltration of T cells within and around the tumor, resulting in a limited therapeutic effect of an anti-PD-1 antibody or an anti-PD-L1 antibody. The medicament prepared from *Christensenella sp.* of the present invention can inhibit the proportion of MDSC in the tumor microenvironment by relieving immunosuppression and effectively inhibit immune cell-suppressive tumor, thereby achieving antitumor use.

The medicament prepared from *Christensenella sp.* provided by the present invention can be used in tumor types for which treatment with PD-1 antibody or anti-PD-L1 antibody is clinically ineffective.

The medicament prepared from *Christensenella sp.* provided by the present invention can significantly promote IFNβ transcriptional activity with efficacy approaching the promotion level of a positive drug (sting agonist) on type I interferon IFNβ. The type I interferon IFNβ has been proved to reconstruct the synergistic effect of innate and adaptive immunity in the tumor microenvironment, making it suitable for treating refractory and drug-resistant cancer. The medicament prepared from *Christensenella sp.* provided by the present invention has an efficacy comparable to that of a sting agonist, that is, confirming its ability to achieve anti-tumor through the immunomodulation, and demonstrating potential utility against viral tumor.

The present invention has experimentally validated the efficacy of the medicament prepared from *Christensenella sp.* alone or in combination with biologics (e.g., anti-PD-1 antibody) or chemotherapeutic drug (e.g., gemcitabine), in three clinically refractory tumor types: liver cancer, lung cancer, and pancreatic cancer, which can achieve effective inhibition of tumor growth, reduction of the tumor volume and/or tumor weight; and improvement of the tumor immune response rate.

BRIEF DESCRIPTION OF THE DRAWINGS

In order to more clearly illustrate the specific embodiments of the present invention, or the technical solutions in the prior art, the drawings to be used in the description of the specific embodiments or the prior art will be briefly introduced below. It will be obvious that the drawings in the following description pertain to some embodiments of the present invention, and for the person of ordinary skill in the art, other drawings can be obtained according to these drawings without creative effort.

FIG. 1 shows a histogram of the tolerance at different pH values of *Christensenella sp.* MNH04863 provided in Example 1 of the present invention.
FIG. 2 shows a histogram of the tolerance at different concentrations of NaCl of *Christensenella sp.* MNH04863 provided in Example 1 of the present invention.
FIG. 3 shows a histogram of the bile salt tolerance of *Christensenella sp.* MNH04863 provided in Example 1 of the present invention.
FIG. 4 shows a phylogenetic tree of *Christensenella sp.* MNH04863 provided in Example 2 of the present invention.
FIG. 5 shows a schematic diagram of the expression of chemokines and inflammatory factors induced in macrophages after co-culture of macrophages and MNH04863 provided in Example 5 of the present invention.
FIG. 6 shows a schematic diagram of the increased chemokine expression in primary peripheral blood mononuclear cells induced by MNH04863 provided in Example 6 of the present invention.
FIG. 7 shows a curve graph of mouse tumor volume in the lung cancer tumor validation experiment provided in Example 7 of the present invention. Data are presented as mean $\pm$ standard deviation (Mean $\pm$ SD), and statistical analysis was performed using two-way ANOVA with Sidak's multiple comparisons test. Significant differences are indicated by *, and *$p < 0.05$.
FIG. 8 shows a graph comparing the tumor volume of mice at the experimental endpoint in the lung cancer tumor validation experiment provided in Example 7 of the present invention. Data are presented as mean $\pm$ standard deviation (Mean $\pm$ SD), and statistical analysis was performed using Student's t-test. Significant differences are indicated by *, *$p < 0.05$, and **$p < 0.01$; and no significance is indicated if not marked.
FIG. 9 shows a graph comparing the tumor weight of mice at the experimental endpoint in the lung cancer tumor validation experiment provided in Example 7 of the present invention. Data are presented as mean $\pm$ standard deviation (Mean $\pm$ SD), and statistical analysis was performed using Student's t-test. Significant differences are indicated by *, *$p < 0.05$, and **$p < 0.01$; and no significance is indicated if not marked.
FIG. 10A and FIG. 10B respectively show a graph of comparison of tumor inhibition rate of mice (FIG. 10A) and a curve graph of tumor inhibition rate of mice (FIG. 10B) at the experimental endpoint in the lung cancer tumor validation experiment provided in Example 7 of the present invention. Data are presented as mean $\pm$ standard deviation (Mean $\pm$ SD), and statistical analysis was performed using Student's t-test. Significant differences are indicated by *, *$p < 0.05$, and **$p < 0.01$.
FIG. 11A and FIG. 11B respectively show graphs of response rates of mice in Group A (FIG. 11A) and Group B (FIG. 11B) at the experimental endpoints in the lung cancer tumor validation experiment provided in Example 7 of the present invention, where the data is shown as individual mouse tumor volumes ($mm^3$).
FIG. 12 shows a graph of tumor volume of mice treated with a combination with PD-1 antibody in a lung cancer tumor model provided in Example 8 of the present invention. Data are presented as mean $\pm$ standard deviation (Mean $\pm$ SD), and statistical analysis was performed using two-way ANOVA with Sidak's multiple comparisons test. Significant differences are indicated by *, and *$p < 0.05$.
FIG. 13 shows a graph of the response rate of mice at the experimental endpoint in a therapeutic experiment in combination with a PD-1 antibody in a lung cancer tumor model provided in Example 8 of the present invention, where the data is shown as individual mouse tumor volumes ($mm^3$).
FIG. 14 shows a graph of the volume change of tumor of experimental mice in the liver cancer tumor experiment provided in Example 9 of the present invention. Data are presented as mean $\pm$ standard deviation (Mean $\pm$ SD), and statistical analysis was performed using two-way ANOVA with Sidak's multiple comparisons test. Significant differences are indicated by *, and *$p < 0.05$.
FIG. 15 shows a graph comparing the tumor suppression rate of experimental mice in the liver cancer tumor experiment provided in Example 9 of the present invention. Data are presented as mean $\pm$ standard deviation (Mean $\pm$ SD), and statistical analysis was performed using Student's t-test. Significant differences are indicated by *, *$p < 0.05$, and **$p < 0.01$.
FIG. 16 shows a graph of the tumor volume of mice in the treatment in combination with gemcitabine in tumor of pancreatic cancer mice provided in Example 12 of the present invention. Data are presented as mean $\pm$ standard deviation (Mean $\pm$ SD), and statistical analysis was performed using two-way ANOVA with Sidak's multiple comparisons test. Significant differences are indicated by *, and *$p < 0.05$.

FIG. 17 shows a graph comparing the tumor volume of mice at the experimental endpoint in combination with gemcitabine in tumor of pancreatic cancer mice provided in Example 12 of the present invention. Data are presented as mean ± standard deviation (Mean ± SD), and statistical analysis was performed using one-way ANOVA with Dunnett's multiple comparisons test. Significant differences are indicated by *, *p < 0.05, and **p < 0.01; and no significance is indicated if not marked.

FIG. 18 shows a graph comparing the tumor weight of mice at the experimental endpoint in combination with gemcitabine in tumor of pancreatic cancer mice provided in Example 12 of the present invention. Data are presented as mean ± standard deviation (Mean ± SD), and statistical analysis was performed using one-way ANOVA with Dunnett's multiple comparisons test. Significant differences are indicated by *, *p < 0.05, and **p < 0.01; and no significance is indicated if not marked.

FIG. 19 shows a graph comparing the tumor inhibition rate of mice at the experimental endpoint with a combination with gemcitabine in tumor of pancreatic cancer mice provided in Example 12 of the present invention. Data are presented as mean ± standard deviation (Mean ± SD), and statistical analysis was performed using one-way ANOVA with Dunnett's multiple comparisons test. Significant differences are indicated by *, *p < 0.05, and **p < 0.01.

FIG. 20 shows a graph of the tumor volume of mice in an experiment in combination with PD-1 antibody in tumor of pancreatic cancer mice provided in Example 13 of the present invention. Data are presented as mean ± standard deviation (Mean ± SD), and statistical analysis was performed using two-way ANOVA with Sidak's multiple comparisons test. Significant differences are indicated by *, and *p < 0.05.

FIGs. 21A to 21C show graphs of response rates of mice at the experimental endpoint in experiment in combination with PD-1 antibody in tumor of pancreatic cancer mice provided in Example 13 of the present invention, where the data is shown as individual mouse tumor volumes (mm$^3$).

FIG. 22A and FIG. 22B respectively show the analysis of the proportion of MDSC in tumor tissue (FIG. 22A) and the correlation analysis of MDSC (Myeloid-derived suppressor cells) with the efficacy of the drug (FIG. 22B) in the mouse hepatocellular carcinoma model.

FIGs. 23A to 23C show the effect of MNH04863 on T cells in tumor tissue during lung cancer treatment. FIG. 23A: CD4/CD45%, FIG. 23B: CD8/CD45% and FIG. 23C: Foxp3/CD4% ratio analysis.

FIG. 24 shows the results of the proportion of IFN-$\gamma^+$ and TNF-$\alpha^+$ cells in CD4 and CD8 cells of tumor tissue in a lung cancer mouse model treated in combination with PD-1 antibody.

FIG. 25 shows the correlation analysis between the effect of MNH04863 & PD-1 on the proportion of tumor immune cells and tumor weight.

FIG. 26 shows the effect of MNH04863 on IFNβ transcriptional activity.

DETAILED DESCRIPTION OF THE EMBODIMENTS

The technical solutions of the present invention will be clearly and completely described below in connection with examples, and it is evident that the examples described are a part of the examples of the present invention and not all of the examples. Based on the examples of the present invention, all other examples obtained by a person of ordinary skill in the art without making creative effort shall fall within the scope of protection of the present invention.

Unless otherwise defined, all technical and scientific terms used in the present invention have the same meanings as commonly used in the field to which the present invention belongs. For the purpose of interpreting this description, the following definitions will be applied and, where appropriate, terms used in the singular form will also include the plural form and vice versa.

The singular form "a" and "an" or any singular designation as used herein include plural reference unless the context clearly indicates otherwise.

The *Christensenella sp.* MNH04863 used in the present invention was originally isolated from a fecal sample of a Han Chinese healthy male volunteer in Guangzhou City, Guangdong Province, and was deposited on August 4, 2020 at the Guangdong Microbial Culture Collection Center. The deposit destigate is *Christensenella sp.* MNH04863, with a deposit number of GDMCC NO: 61117; the address is Guangdong Institute of Microbiology, 5th Floor, Building 59th, 100, Xianlie Middle Road, Guangzhou, Guangdong, and the viability test result is survival. The taxonomic name is *Christensenella sp..* The strain has been disclosed in patent document, such as CN113069475A.

Exemplary characteristics of the strain are listed below:

(1) The 16s rRNA sequence of the *Christensenella sp.* MNH04863 is as shown in SEQ ID NO:1.

(2) The physiological and biochemical characteristics of the the the *Christensenella sp.* MNH04863 are as follows: *Christensenella sp.* MNH04863 grows at a temperature range of 30~42°C, and it can grow in the range of pH 6.0~10.0; it can tolerate up to 2% NaCl; it can survive and grow in the concentration range of 0~0.15% of bile salt, and it can't grow in the case of concentration ≥0.4% of bile salt; it can't grow under aerobic conditions, and it grows well under anaerobic conditions, and it belongs to an obligatory anaerobic bacteria.

(3) the *Christensenella sp.* MNH04863 is a Gram-negative bacterium, specifically Gram-stain-negative C. *intestinihominis*.

(4) the *Christensenella sp.* MNH04863 has an ANI value of ≤ 83.5% with other strains within the genus *Christensenella*.

As used herein, "metabolite" refers to a substance used as substrate or product compound, composition, or molecule in any cellular or microbial metabolic reaction, or an ion, cofactor, catalyst, or nutrient derived from a cellular or microbial metabolic reaction.

"Strain" refers to a member of a bacterial species with genetic characteristics that make it distinguishable from closely related members of the same bacterial species. Genetic characteristics can be the complete or partial absence of at least one gene, the complete or partial absence of at least one regulatory region (e.g., promoter, terminator, riboswitch, ribosome-binding site), the absence of at least one natural plasmid ("cure"), the presence of at least one recombinant gene, the presence of at least one mutated gene, the presence of at least one exogenous gene (a gene from another species), the presence of at least one mutated regulatory region (e.g., promoter, terminator, riboswitch, ribosome-binding site), the presence of at least one unnatural plasmid, the presence of at least one antibiotic resistance cassette, or any combination thereof. Genetic characteristics between different strains can be identified by PCR amplification, optionally followed by DNA sequencing of the genomic region of interest or the whole genome. In cases where a strain (compared to another strain of the same species) gains or loses antibiotic resistance or gains or loses biosynthetic capacity (e.g., nutrient-deficient strains), strain or nutrient/metabolite can be distinguished by selection or counter-selection using antibiotics.

"Supernatant liquid" or "supernatant" of the present invention refers to a culture supernatant of a bacterial strain according to the present application, optionally comprising compounds and/or cellular debris of the strain, and/or metabolites and/or molecules secreted by the strain.

The medicament of the present application may be formulated in any form suitable for enhancing the abundance of *Christensenella sp.* in a subject. In some embodiments, the medicament is administered orally (e.g., by oral gavage). In some embodiments, the medicament may be administered via various routes including intramuscular injection, inhalation, intracranial, intralymphatic, intraocular, intraperitoneal, intrapleural, intrathecal, intratracheal, intrauterine, intravascular, intravenous, intravesical, intranasal, gastrointestinal, biliary perfusion, cardiac perfusion, pre-anal, rectal, spinal subcutaneous, sublingual, topical, intravaginal, transdermal, ureteral, or urethral and the like. Examples of applicable dosage forms include, but are not limited to, tablets, aerosols, chewable sticks, capsules, capsules containing coated particles, capsules containing sustained-release particles, capsules containing sustained-release particles, concentrates, creams, enhanced creams, suppositories, disks, dressings, elixirs, emulsions, enemas, sustained-release fibers, sustained-release films, gases, gels, metered gels, granules, sustained-release granules, powders, sustained-release powders, metered powders, ring, shampoo, soap solution, slurry solution, solution/drops, concentrated solution, gel forming solution/drops, effervescent tablets, chewing gum, implants, inhalers, injections, lipid complex injections, liposome injection, inserts, sustained-release inserts, intrauterine devices, jellies, liquids, sustained-release liquids, lotions, enhanced lotions, oils, ointments, enhanced ointments, paste, sponges, sprays, metered sprays, suppositories, suspensions, suspensions/drops, sustained-release suspensions, swabs, syrups, tablets, chewable tablets, tablets containing coated granules, dispersible tablets, effervescent tablets, sustained-release tablets, orally disintegrating tablets, tampons, tapes, or cannulas/troches. In some embodiments, the medicament is a sugar-coated tablet, gel capsule, gel, emulsion, tablet, tablet capsule, hydrogel, nanofiber gels, electrospun fiber, food bar, candy, fermented milk, fermented cheese, chewing gum, powder, or toothpaste and the like. In some embodiments, the medicament may be administered by inclusion in the subject's diet, such as inclusion in a functional food for use in humans or companion animals. As used in the present invention, a subject may be a human or animal, including but not limited to a cow, a sheep, a cat, a canine, a horse, a rabbit, a monkey, a mouse, a rat, an alpaca, a camel, and the like.

In some embodiments, the composition of the present application is administered orally. Oral administration may involve swallowing, whereby the compound enters the gastrointestinal tract, and/or oral, lingual or sublingual administration, whereby the compound is absorbed directly into the bloodstream from the oral through the oral mucosa. Pharmaceutical dosage forms suitable for oral administration include solid suppositories, solid microparticles, semisolids and liquids (including multiphase or dispersible systems) e.g. tablets; soft or hard capsules containing multiple particles or nanoparticles, liquids (e.g., aqueous solutions), emulsions, or powders; troches (including liquid filler); chewables; gels; fast-dispersing dosage forms; ovules; sprays; and buccal/mucosal adhesion patches.

In some embodiments, the composition is an enteric formulation, i.e., gastric fluid-tolerant formulations (e.g., tolerant to gastric pH) suitable for delivery of the composition of the present application to the intestines by oral administration. The enteric formulation may be particularly useful when the bacterium or another component of the composition is acid-sensitive, for example readily degradable under gastric conditions.

In some embodiments, the enteric formulation comprises an enteric coating. In some embodiments, the composition is in an enteric-coated dosage form. For example, the composition may be an enteric-coated granule, enteric-coated tablet or enteric-coated capsule and the like. Enteric-coated granules refer to particles coated with enteric materials or prepared by other suitable methods. Enteric-coated granules are resistant to gastric acid and release active ingredients in the intestinal fluid or control the specific location-release of the drug within the intestine, which can prevent the drug from decomposing or becoming ineffective in the stomach, thereby avoiding irritation to the stomach. Enteric-coated tablets refer to tablets coated with enteric coating materials to prevent the API (Active Pharmaceutical Ingredient) from decomposing or becoming ineffective in the stomach, to avoid irritation to the stomach, or to control the specific location-release of API within the intestine. The tablets can be coated with a colon-specific enteric coating to ensure specific-release for the treatment of diseases of the colon. Enteric-coated capsules refer to hard capsules filled with enteric-coated granules or pellets, or they are prepared using suitable enteric coating materials to produce either hard or soft capsules. Enteric-coated capsules are insoluble in gastric fluid but can disintegrate in intestinal fluid to release the active ingredients.

In some embodiments, the composition is a soft capsule formulation. Soft capsules refer to capsules in which a certain amount of a liquid API is sealed directly, or a solid API is dissolved or dispersed in a suitable excipient to be prepared as a solution, suspension, emulsion, or semi solid formulations, and are sealed in a soft capsule material. The flexibility and elasticity of soft capsules result from the addition of softeners, such as glycerol, sorbitol, maltitol, and polyethylene glycol in the capsule shell. Soft capsules can be produced, for example, on the basis of gelatin or starch. Gelatin-based soft capsules are commercially available by purchasing from a variety of suppliers. Depending on the method of administration, e.g., oral or rectal administration, the soft capsules may have various shapes, such as, round, oval, oval or torpedo shaped. Soft capsules can be produced by conventional processes, for example by the Scherer process, the Accogel process, or a droplet or blow molding process.

In some embodiments, the composition is a microencapsulated capsule where the microencapsulation protects the composition from degradation until it is delivered to the target site. For example, it may be released upon chemical or physical stimuli such as pressure, enzymatic activity, or physical disintegration, which may be triggered by a change in pH. Any suitable encapsulation method can be used. Exemplary encapsulation techniques include entrapment within a porous matrix, attachment or adsorption on the surface of a solid carrier, self-aggregation through flocculation or the use of cross-linking agents, and mechanical containment behind a microporous membrane or within microencapsulated capsule.

In some embodiments, the composition is a capsule formulation. A capsule refers to a solid dosage form prepared by filling the API or with suitable excipients in a hollow capsule or sealed in a soft capsule shell. Capsules can be categorized into hard capsules and soft capsules. There are also sustained-release capsules, controlled-release capsules, and enteric-coated capsules depending on the release characteristics. Hard capsule (commonly known as capsule) refers to the use of appropriate preparation technology, filling uniform powder, granules, small tablets, small pellets, semi-solid or liquid, etc. prepared by API or API with suitable excipients into the hollow capsule. Hard capsules is formulated by filling into hollow capsules with different forms of contents prepared according to the following formulation techniques: (1) preparing uniform powders, granules or small tablets by mixing API and suitable excipients such as diluents, giluents, disintegrating agents, etc.; (2) filling ordinary pellets, immediate-release pellets, sustained-release pellets, controlled-release pellets, or enteric-coated pellets individually or in combination, and if necessary, adding an appropriate amount of blank small pellets as a filler; (3) filling powder of API directly; (4) preparing API as inclusion compounds, solid dispersions, microcapsules or microspheres; (5) filling solutions, suspensions, emulsions, etc. into hollow capsules using a specialized capsule-filling machine, followed by sealing if necessary. Soft capsule refers to a capsule in which a certain amount of liquid API is directly sealed, or solid API is dissolved or dispersed in suitable excipients to prepare into a solution, suspension, emulsion or semi-solid, encapsulated in a soft capsule shell material. It can be prepared by the drop method or by the press method. A soft capsule shell material is generally made from gelatin for capsule, glycerin, or other suitable pharmaceutical excipients for capsules, either alone or in blends. Sustained-release capsule is a capsule that slowly release drug at a non-constant rate in a specified release medium. A controlled release capsule is a capsule that slowly releases drug at a constant rate in a specified release medium. Enteric-coated capsule refers to a hard capsule prepared by filling a capsule shell with enteric-coated granules or pellets, or a hard capsule or soft capsule prepared with an appropriate enteric material. Enteric-coated capsules are insoluble in gastric fluid but can disintegrate in intestinal fluid to release the active ingredients.

In some embodiments, the composition is prepared by freeze-drying or spray-drying. Freeze-drying method is to cool down and freeze a liquid drug into a solid frozen state, then sublimation drying under vacuum environment to remove ice crystals. After sublimation, the same is placed under vacuum environment for desorption drying to remove the bound water. Spray drying is a drying method that uses a micromization device to atomize the solution into small droplets, and then the small droplets come into contact with a relatively high temperature drying air, thus evaporating the water. This method is widely used in the food, pharmaceutical and chemical industries for products of different properties. Spray drying method can be electrostatic spray drying method. Electrostatic spray method makes water or other solvents in spray drying atomized droplets under the electrostatic force effect to repel each other and accumulate at the edge of the droplets, while the core of the solid components are retained in the center of the droplets, thus reducing the difficulty of drying and evaporation of water, that is, reducing the temperature required for water evaporation, minimizing the loss, degradation, or denaturation of core active ingredients due to high temperatures.

As used herein, the composition comprises a pharmaceutical composition, health care product or food product.

The pharmaceutical composition provided by the present invention may comprise an adjuvant. Suitable pharmaceutically acceptable adjuvant that may be used include, for example, carrier, excipient, diluent, lubricant, wetting agent, emulsifier, suspension stabilizer, preservative, sweetener, and flavor. For example, pharmaceutically acceptable adjuvants are one or more of lactose, glucose, sucrose, sorbitol, mannose, starch, acacia, calcium phosphate, alginate, gelatin, calcium silicate, finely crystalline cellulose, polyvinylpyrrolidone, cellulose, water, syrup, methyl cellulose, methyl hydroxybenzoate, propyl hydroxybenzoate, talc, magnesium stearate and mineral oil.

The composition of the present invention may comprise a pharmaceutically acceptable excipient, diluent or carrier. Acceptable carriers or diluents for therapeutic use are well known in the pharmaceutical field. Examples of suitable carriers include lactose, starch, glucose, methylcellulose, magnesium stearate, mannitol, or sorbitol and the like. Examples of suitable diluents include ethanol, glycerol and water. The selection of pharmaceutical carrier, excipient or diluent can be based on the intended route of administration and standard pharmaceutical practice. The pharmaceutical composition may comprise any suitable binder, lubricant, suspending agent, coating agent, or solubilizer as a carrier, excipient, or diluent, in addition to or besides the carrier, excipient, or diluent. Examples of suitable binders include starch, gelatin, natural sugars, and natural or synthetic gums. Wherein the natural sugar is, for example, glucose, anhydrous lactose, free-flowing lactose, β-lactose or corn sweetener. Natural or synthetic gums include such as gum arabic, tragacanth, sodium alginate, carboxymethyl cellulose or polyethylene glycol. Examples of suitable lubricants include sodium oleate, sodium stearate, magnesium stearate, sodium benzoate, sodium acetate, or sodium chloride and the like. Preservatives, stabilizers, dyes and even flavoring agents can be provided in the pharmaceutical compositions. Examples of preservatives include sodium benzoate, sorbic acid or parabens. An antioxidant and suspending agent may be used. An antioxidant and suspending agent may be used.

Depending on the disease and patient to be treated and the route of administration, the pharmaceutical compositions or pharmaceutical formulations of the present invention may be administered once daily or multiple times daily at different doses. The dosage to be administered depends on many factors, such as the severity of the disease to be treated or prevented, the patient's sex, age, body weight and individual response, the route of administration and the frequency of dosing. The above dosage may be administered in a single dose form or divided into several dose forms. e.g., two, three, or four doses.

The actual dosage level of the primary drug in the pharmaceutical formulations of the present invention may be varied so that the desired therapeutic response can be effectively tailored to the specific patient, composition and mode of administration. Dosage levels should be selected based on the route of administration, the severity of the condition being treated, and the condition and medical history of the patient to be treated. However, it is the practice in the field to start with a dosage below the level required to obtain the desired therapeutic effect and gradually increasing the dosage until the desired effect is obtained.

As used herein, the term "subject" or "patient" refers to any mammal. A subject or patient described as "in need" refers to a person who needs treatment (or prevention) of a disease. Mammals (i.e., mammalian animals) include humans, laboratory animals (e.g., primates, rats, mice), livestock (e.g., cattle, sheep, goats, pigs), and household pets (e.g., canines, cats, rodents). The subject can be a person. The subject may be a non-human mammal including, but not limited to, canines, cats, cattle, horses, pigs, donkeys, goats, camels, mice, rats, guinea pigs, sheep, camels, monkeys, gorillas, or chimpanzees. The subject or patient may be healthy or may have a metabolic disease at any stage of development.

As used herein, the term "treating" a subject's disease or "treating" a subject suffering or suspected of suffering from a disease refers to administering medication to the subject, such as administering one or more pharmaceutical agents, which reduces or prevents the worsening of at least one symptom of the disease. Accordingly, in one embodiment, "treating" refers, in particular, to delaying progression, accelerating remission, inducing remission, increasing remission, accelerating recovery, increasing the efficacy of, or decreasing resistance to, alternative therapies, or a combination thereof.

As used herein, the term "prevention" is recognized in the art and is well known in the art when used in connection with conditions such as localized recurrence, and includes administration to reduce the occurrence or delay the onset of symptoms of a medical condition in a subject relative to a subject not receiving the composition. Accordingly, cancer prevention comprises, for example, reducing the number of detectable tumors in a population of patients receiving preventive treatment relative to an untreated control population, and/or delaying the appearance of detectable tumors in a treated population relative to an untreated control population by, for example, a statistically and/or clinically significant amount.

As used herein, "enhancing" the efficacy of a cell therapy (e.g., chimeric antigen receptor T-cell (CAR-T) therapy) refers to that the composition of the present invention improves the therapeutic effect deriving from a cell therapy (e.g., in the case of CAR-T, a T-cell-mediated immune response, in particular, against a tumor antigen) as a result of its administration, when compared to no administration. For example, a subject treated with a composition of the present invention and cellular therapy may exhibit greater such therapeutic effects from cell therapy than a control subject treated with cell therapy rather than a composition of the present invention.

As used herein, "probiotic" refers to live bacteria or yeasts that, when consumed, are beneficial to the health of the host, and are to restore the balance of intestinal bacteria that have been disrupted by long-term use of antibiotics or gastrointestinal disorders.

As used herein, "effective amount" refers to an amount that is therapeutically or prophylactically effective for the subject being treated. The precise amount of the therapeutic composition also depends on the judgment of the practitioner and is likely to be unique for each individual. The suitable regimen for initial administration and booster is also variable, but may be represented by initial and subsequent administration. Factors affecting dosage include the physical and clinical status of the patient, the route of administration, the intended therapeutic goal (symptomatic relief or cure) and the efficacy, stability, and toxicity of the particular therapeutic substance or the efficacy of other treatments that the subject may be undergoing.

Excessive cell proliferation can lead to the development of tumors or neoplasms. When tumor cells acquire the ability to invade surrounding tissues, for example, by rupturing and entering the bloodstream or lymphatic system, or forming secondary tumors in other parts of the body, they are considered cancerous. Thus, as used herein, "tumor" includes both benign and malignant tumors (i.e., cancers). In some cases, "tumor" and "cancer" are used interchangeably to refer to the presence of cells exhibiting typical characteristics of oncogenic cells, including but not limited to uncontrolled proliferation, immortalization, metastatic potential, rapid growth and proliferation rates, and certain characteristic morphological features.

Without wishing to be limited by theory, it is believed that the microbial agents or medicament of the present invention are capable of immunomodulating macrophages and/or primary PBMC cells and/or monocyte-derived dendritic cells, and thereby inhibiting the growth of the tumors.

Recent studies have shown that the intestinal microbes of cancer patients significantly influence the efficacy of tumor immunotherapy, and modulating and utilizing intestinal microbes has become a key component of tumor immunotherapy.

It has been demonstrated that multiple mechanisms of direct interaction exist between intestinal microbes and immune cells, and dendritic cells and macrophages in intestine-associated lymphoid tissue are key target cells for immunomodulatory microbes, particularly in the small intestine, where the large surface area and thin, diffuse mucus layer allow microbes to closely interact with immune cells. Different specific strains within the microbial flora can suppress or activate immune responses throughout the body.

Macrophages are a heterogeneous population of myeloid cells in the innate immune system that participate in various physiological and pathological processes. During inflammation or infection in organism, blood monocytes migrate into tissues and differentiate into macrophages. Macrophages exhibit high plasticity and primarily exist in two polarized states: classically activated type 1 (M1) and alternatively activated type 2 (M2). M1 macrophages are pro-inflammatory cytokine-producing macrophages, known as classical type macrophages, which typically emerge following injury or infection. *In vitro,* classical activation of macrophages is commonly induced by bacterial cell wall components (e.g., LPS) together with TNF-$\alpha$ or IFN-y. M1 macrophages are characterized by secreting pro-inflammatory cytokines such as TNF-$\alpha$, IL-1$\beta$, IL-6, IL-12 and IL-8, playing important roles in the early stages of inflammation. M2 macrophage polarization can be induced by various stimuli, primarily IL-4 and/or IL-13. Macrophages activated by IL-4 and IL-13 secrete anti-inflammatory cytokines such as IL-10, CCL18 and CCL22, functioning to suppress inflammatory responses and promote tissue repair.

M1 macrophages exhibit cytotoxicity against both pathogens and tumor cells. Their anti-tumor activity is associated with their ability to secrete reactive nitro group species, reactive oxygen species and pro-inflammatory cytokines. M2 macrophages promote tumor cell growth and survival by secreting various growth factors such as EGF, TGF-$\beta$ or VEGF. Therefore, detecting the induction of pro-inflammatory and anti-inflammatory cytokine expression in macrophages by different bacterial strains can serve as an indicator for assessing their potential anti-tumor properties.

The composition of the present invention can also inhibit tumors through short-chain fatty acids or short-chain fatty acid salts, thereby achieving anti-tumor applications. short-chain fatty acids (SCFAs) are lipid substances composed of 2-6 carbon atoms, including acetate, propionate and butyrate, which are primarily produced by intestinal microbial fermentation of dietary fiber. Recent studies have shown their potential anti-cancer effects in various extraintestinal cancers including bladder cancer, breast cancer, gastric cancer, liver cancer, lung cancer, pancreatic cancer and prostate cancer (doi: 10.1016/j.biopha.2021.111619). SCFAs can be specifically recognized by G protein-coupled receptors (GPCRs) on cancer cell surfaces. Additionally, due to their small molecular size, SCFAs can enter the nuclei of cancer cells and inhibit histone deacetylase (HDAC) activity, thereby upregulating histone acetylation and crotonylation to exert direct anti-cancer effects. SCFAs can also indirectly exert anti-cancer effects by modulating immune cells: intestinal bacteria produce SCFAs through dietary fiber fermentation, which can not only act locally in the intestine but also reach the liver and systemic circulation through the portal vein system to exert anti-cancer effects; after being recognized by GPCRs on cancer cell surfaces, SCFAs promote cancer cell apoptosis, cell cycle arrest and inhibit cancer cell metastasis by increasing TNF-$\alpha$ secretion, upregulating Fas-L expression, reducing Bcl-2 expression, downregulating CDK and Cyclin activity, and inhibiting the MAPK/ERK pathway. Upon entering cancer cell nuclei, SCFAs inhibit HDAC, thereby increasing TNF-$\alpha$ secretion, upregulating Fas-L expression, reducing oxygen consumption or lactate metabolism; downregulating Cyclin activity while upregulating expression of the CDK inhibitor p21 gene; and inhibiting the Akt/ERK pathway, thereby promoting cancer cell apoptosis, cell cycle arrest and inhibiting cancer cell metastasis. SCFAs can enhance the ability of B cells secreting IgA and IgG, enhance the ability of T cells secreting IFN-y, increase MICA/B expression on cancer cells to improve recognition ability of NK cell, and enhance the ability of macrophages producing ROS (doi: 10.12354/j.issn.1000-8179.2022.20211821). Therefore, the microbial agent or medicament of the present invention is expected to inhibit tumors through SCFAs.

The composition of the present invention can also achieve anti-tumor effects by relieving immunosuppression, particularly where the microbial agent or composition can be served as an immunomodulator for myeloid-derived suppressor cells (MDSCs) in cancer patients. Myeloid-derived suppressor cells (MDSCs) are a population of heterogeneous myeloid cells represented by bone marrow progenitors and immature granulocytes, dendritic cells and macrophages, which express diverse antigen markers distinct from mature myeloid cells. MDSCs are morphologically and phenotypically similar to neutrophils and monocytes, but differ from other myeloid cell populations in their immunosuppressive functions. Compared to mature neutrophils and monocytes, MDSCs are more readily generated under pathological conditions and can be activated by various factors including interleukin-13 (IL-13), IL-14 and interferon-y (IFN-y). Significantly elevated MDSC levels have been detected in the TME and peripheral blood of patients with nearly all cancer types, including melanoma, glioma, head and neck neoplasm, lung cancer, colorectal cancer, bladder cancer, nasopharyngeal carcinoma, liver cancer, kidney cancer, pancreatic cancer and lymphoma. The quantity and activity of MDSCs impact patient prognosis (Jianjiao NI. Molecular Mechanisms and Clinical Significance of STING Pathway Regulation of Myeloid-Derived Suppressor Cells in Nasopharyngeal Carcinoma [D]. Peking Union Medical College, Chinese Academy of Medical Sciences [2023-12-26]). Currently, it is known that myeloid-derived suppressor cells (MDSCs) can inhibit anti-tumor immune responses through various mechanisms. Among the most important are the production of reactive oxygen species (ROS), inducible nitric oxide synthase (iNOS) and high levels of arginase-1 (Arg-1), which directly suppress the activation and expansion of T cells. In addition to these mechanisms, MDSCs can also modulate the body's immune suppression by upregulating transforming growth factor (TGF), IL-10, etc., and regulating the expression of regulatory T cells (Treg cells). MDSCs have now gained widespread attention, with the recognition that targeting MDSCs represents an important direction for clinical cancer immunotherapy. Current MDSC-targeted therapeutic strategies of tumors mainly focus on: suppressing MDSC immunosuppressive functions, and inhibiting MDSC proliferation, recruitment and differentiation. Therefore, the microbial agent, medicament or composition of the present invention is expected to exert anti-tumor effects by reducing MDSC proportions in the tumor microenvironment.

The compositions of the present invention are also capable of preventing or treating tumors by promoting IFN$\beta$ transcriptional activity. Type I interferon IFN$\beta$ has been demonstrated to restore the synergistic interaction between innate and adaptive immunity within the tumor microenvironment, and is used in the treatment of refractory drug-resistant cancers (see "Targeting the Tumor Microenvironment with Interferon-$\beta$ Bridges Innate and Adaptive Immune Responses," Yang X1, Cancer Cell, 2014, doi: 10.1016/j.ccr.2013.12.004). The medicament prepared from *Christensenella sp.* of the present invention can significantly promote IFN$\beta$ transcriptional activity, with efficacy close to that of the positive drug (a STING agonist) in enhancing type I interferon IFN$\beta$ expression. Stimulator of Interferon Genes (STING), also known as MITA, MPYS, ERIS, and TMEM173, was first identified in 2008. It is a 28 kD endoplasmic reticulum (ER)-localized dimeric adaptor protein composed of 379 amino acids. The protein includes an N-terminal domain that folds into four transmembrane helices, a central globular domain, and a cytoplasmic C-terminal tail. The central globular domain is comprised within the C-terminal domain, and STING protein anchors to the endoplasmic reticulum through several transmembrane domains located in its N-terminal region. STING transduces signals from upstream DNA sensors to downstream signaling molecules such as TBK1/IRF3 and NF-$\kappa$B, ultimately inducing the expression of type I interferons (IFNs) and pro-inflammatory cytokines. Therefore, STING as the innate immune system, not only regulates IFN produced by viral or bacterial infection but also participates in the induction of anti-tumor immune responses. STING agonists have been demonstrated to be critical for regulating anti-viral responses and anti-tumor adaptive immunity and are being widely developed as a new class of cancer therapeutics. Therefore, it is expected that the microbial agent, medicament, or composition of the present invention may exert antiviral and anti-tumor effects by activating the STING signaling pathway and inducing IFN$\beta$ expression.

The following examples and drawings are provided below to aid in understanding the present invention. It should be understood, however, that these examples and drawings are intended to illustrate the present invention only, but do not constitute any limitation on the present invention. The actual scope of protection of the present invention is set forth in the claims. It should be understood that any modifications and changes may be made without departing from the spirit of the present invention.

Examples

The liquid MM01 medium involved in the examples of the present invention has the following components: 5 g/L of peptone, 5 g/L of trypticase, 10 g/L of yeast powder, 5 g/L of beef extract, 5 g/L of glucose, 2 g/L of $K_2HPO_4$, 2 g/L of sodium acetate, 1 mL/L of Tween 80, 5 mg/L of hemoglobin, 0.5 g/L of L-cysteine hydrochloride, 1 $\mu$L/L of vitamin K1, and 8 ml/L of inorganic salt solution (including per liter: 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride).

The solid MM01 medium involved in the examples of the present invention has the following components: 5 g/L of peptone, 5 g/L of trypticase, 10 g/L of yeast powder, 5 g/L of beef extract, 5 g/L of glucose, 2 g/L of $K_2HPO_4$, 2 g/L of sodium acetate, 1 mL/L of Tween 80, 5 mg/L of hemoglobin, 0.5 g/L of L-cysteine hydrochloride, 1 $\mu$L/L of vitamin K1, and 8 ml/L of inorganic salt solution (including per liter: 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride), and 15 g/L of agar.

The AC liquid medium involved in the examples of the present invention, comprises per liter: peptone, 20 g; glucose, 5 g; yeast extract, 3 g; beef extract powder, 3 g; vitamin C, 0.2 g; and pH 7.0.

The anaerobic blood agar plate (purchased from Huankai Microbial) involved in the examples is formulated (per liter) as follows: 10.0 g of casein tryptic digest, 3.0 g of cardiac tryptic digest, 1.0 g of corn starch, 5.0 g of meat pepsin digest, 5.0 g of yeast extract powder, 5.0 g of sodium chloride, 15.0 g of agar, 50-100 mL of sterile defibrinated sheep blood, and 1000 mL of distilled water; and final pH $7.3\pm0.2$.

The tryptic soy broth (TSB) liquid medium involved in the examples of the present invention comprises per liter: 17.0 g of tryptone, 3.0 g of soybean papain hydrolysate, 2.5 g of dipotassium hydrogen phosphate, 5.0 g of sodium chloride, and 2.5 g of glucose; and pH $7.3 \pm 0.2$.

Conventional methods of preparation and sterilization may be used to prepare the above medium.

In the examples of the present invention, the solvent (also referred to as PBS-Cys (glycerol)) is prepared by mixing PBS-Cys (phosphate buffer containing 0.05% of cysteine hydrochloride) with 100% of glycerol at a ratio of 3:1 and thoroughly mixed.

In the examples of the present invention, the Tumor Growth Inhibition rate (TGI) is calculated in the following manner:

Tumor Growth Inhibition rate: (Average volume of the control group - Volume of the experimental group) / Average volume of the control group $\times$ 100%. All data are presented as mean $\pm$ SD, and GraphPad Prism 8.0.2 software is used for plotting and statistical analysis. For pairwise comparisons, the Student's t-test is employed. For two-factor analysis, two-way ANOVA with Sidak's multiple comparisons test is applied. Significant difference is indicated by *, *$p < 0.05$, **$p < 0.01$, ***$p < 0.001$, and ****$p < 0.0001$.

In the examples of the present invention, the response rate is calculated in the following manner:

$$\text{Response rate} = \text{Number of responding mice / Number of surviving mice in the group} \times 100\%;$$

Response criteria: Non-response is defined when the average tumor volume exceeds a 40% inhibition rate, while response is defined when the average tumor volume is less than or equal to a 40% inhibition rate. A 40% inhibition rate = Average tumor volume of the negative control group $\times$ (1 - 40%) $\times$ 100%.

Example 1. Physiological and Biochemical Characteristics of *Christensenella* sp. MNH04863

(1) pH Tolerance:

The bacterial strain was inoculated into MM01 medium, sterilized, and centrifuged, then aliquoted into different centrifuge tubes for later use. The bacterial solution from the tubes was taken and inoculated into MM01 mediums with different pH levels, MM01 mediums different NaCl concentrations, and MM01 mediums different sodium cholate concentrations. Three replicates were set for each concentration. After inoculation, the bacterial solution was mixed well, and 200 $\mu$L of bacterial solution was transferred to a 96-well microplate to measure the absorbance at 600 nm.

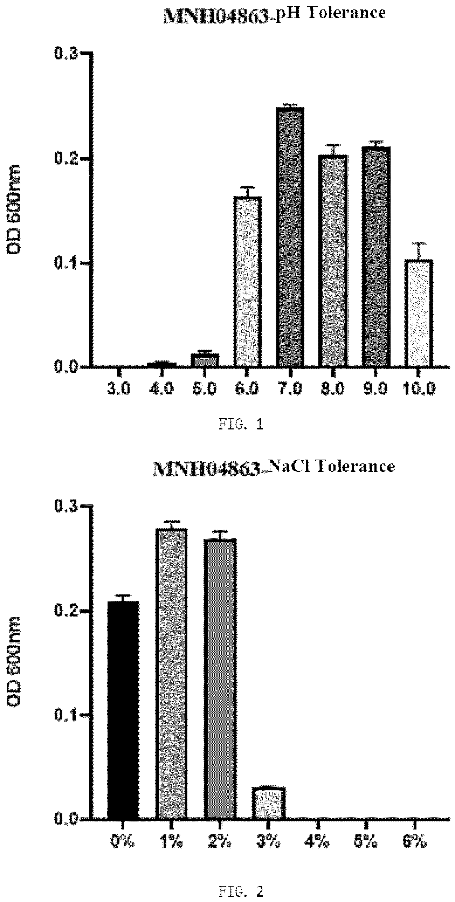

FIG. 1 shows a histogram of the tolerance of *Christensenella sp.* MNH04863 at different pH values in this example.

FIG. 2 shows a histogram of the tolerance of *Christensenella sp.* MNH04863 at different concentrations of NaCl in this example.

(2) Bile Salt Tolerance:

The MM01 medium was aliquoted into 100 mL reagent bottles (100 mL per bottle) 0.10, 0.15, 0.20, 0.20, 0.30 and 0.40 g of sodium cholate were added respectively to prepare MM01 medium with sodium cholate concentrations of 0%, 0.10%, 0.15%, 0.20%, 0.25%, 0.30%, and 0.40%. After sterilization and cooling to room temperature, the medium was aliquoted into 10 mL centrifuge tube (6 mL per tube), with three replicates for each concentration.

0.6 mL aliquot of the first-generation bacterial solution was transferred or a single colony was scraped into 30 mL of MM01 medium, cultured anaerobically at 37°C for 14 to 24 h to obtain the second-generation bacterial solution. 120 μL aliquot of the second-generation bacterial solution was transferred by micropipette and inoculated into 6 mL of MM01 medium containing different bile salt concentrations. After 24 h of incubation, the bacterial solution was pipetted by micropipette to mix well, and 200 μL of bacterial solution was transferred to a 96-well microplate to measure the absorbance at 600 nm.

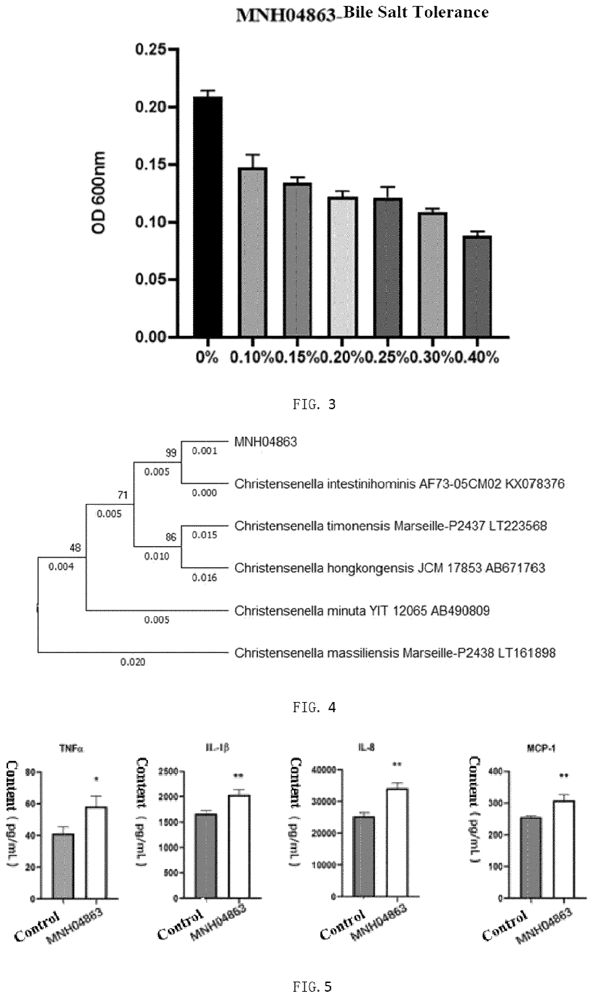

FIG. 3 shows a histogram of the bile salt tolerance of *Christensenella sp.* MNH04863 in this example.

FIGs. 1 to 3 show that *Christensenella sp.* MNH04863 grew at a temperature range of 30~42°C, with an optimal growth temperature of 37°C. It grew in the range of pH 6.0 to 10.0, with an optimal pH of 7.0~9.0. The strain tolerated up to 2% NaCl. *Christensenella sp.* MNH04863 survived and grew at bile salt concentrations of 0~0.15%, but failed to grow at concentrations ≥0.4% (with an OD cutoff of 0.10). The strain did not grow under aerobic conditions but exhibited robust growth under anaerobic conditions, confirming its obligate anaerobic nature.

(3) Antibiotic Susceptibility Testing of *Christensenella sp.* MNH04863:

The disk diffusion method was used to conduct the antibiotic susceptibility testing of *Christensenella sp.* MNH04863. The results are shown in Table 1. *Christensenella sp.* MNH04863 was sensitive to erythromycin, chloramphenicol, tetracycline, penicillin, lincomycin, and ceftriaxone; and *Christensenella sp.* MNH04863 exhibited resistance to ampicillin, compound sulfamethoxazole, ciprofloxacin, and gentamicin.

Table 1: Antibiotic Susceptibility Test Results for *Christensenella sp.* MNH04863:

| Antibiotics | Inhibition Zone Diameter (mm) |
|---|---|
| Ampicillin (AMP) | 0 |
| Compound Sulfamethoxazole (SXT) | 0 |
| Erythromycin (E) | 16.82 |
| Ciprofloxacin (CIP) | 0 |
| Lincomycin (MY) | 29.86 |
| Chloramphenicol (C) | 38.88 |
| Penicillin (PEN) | 43.06 |
| Gentamycin (GEN) | 0 |
| Tetracycline (TET) | 46.11 |
| Ceftriaxone (CTR) | 37.32 |

Example 2. Identification of *Christensenella sp.* MNH04863

I. *Christensenella sp.* MNH04863 was analyzed for 16S rRNA to preliminarily determine their taxonomic classification.

Genomic DNA was extracted from *Christensenella sp.* MNH04863, and the 16S rRNA gene was amplified using the extracted DNA as a template. The amplification primers used were:

27F (5'- AGAGTTTGATCATGGCTCAG-3') (SEQ ID No. 2);
and, 1492R (5'-TAGGGTTACCTTGTTACGACTT-3') (SEQ ID No. 3);

The amplified PCR product was purified and sequenced using the ABI3730XL system, yielding a 1376 bp 16S rRNA sequence. This sequence was aligned with the NCBI database, showing 100% similarity to the closest species *Christensenella intestinihominis,* preliminarily confirming the taxonomic classification of the strain. Thus, MNH04863 was initially identified to belong to *Christensenella intestinihominis.*

According to the report of *Christensenella intestinihominis* in WO2018045493A1, it is known that *Christensenella intestinihominis* strain is Gram-positive, whereas *Christensenella sp.* MNH04863 of the present invention is Gram-negative. Additionally, *Christensenella intestinihominis* in WO2018045493A1 tolerated up to 3% bile salt, whereas *Christensenella sp.* MNH04863 of the present invention can survive and grow only at concentration range of 0~0.15% of bile salt and fail to grow at concentration ≥0.4% of bile salt. Significant differences were also observed in substrate utilization such as glycerol, raffinose, maltose, gelatin hydrolysis, mannitol, esculin hydrolysis, and melezitose. Therefore, the strain in the present invention exhibited distinct characteristics compared to *Christensenella intestinihominis* reported in WO2018045493A1.

II. The isolated *Christensenella sp.* MNH04863 original strain was performed by preparation, sequencing, assembly, and analysis of the genome.

The genomic DNA of *Christensenella sp.* MNH04863 original strain was fragmented by ultrasonication, with a fragment length range of ~350 bp. A Illumina sequencing libraries was constructed using a standard DNA library preparation kit (NEB UltraTM). The constructed libraries were sequenced using NovaSeq (Illumina) with paired-end 150 bp reads.

The genomic original sequencing data were filtered using fastp (version: 0.20.0). Filtered original data were then assembled into a genome using SPAdes (version: v3.14.0).

Gene prediction and annotation of the genome were performed using the Prokaryotic Genome Annotation Pipeline prokka (version: 1.14.5).

Genome information was collected from NCBI:

*Christensenella massiliensis* Marseille-p2438 genome information GCA_900155415.1;
*Christensenella minuta* DSM 22607 genome information GCA_001652705.1; and
*Christensenella timonensis* Marseille-P2437 genome information GCA_900087015.1.

The genome of Christensenella sp. MNH04863 was aligned with those of Christensenella massiliensis, Christensenella minuta, and Christensenella timonensis, and the results are shown in Table 2.

Table 2: Genomic alignment analysis of strains of *Christensenella sp.* MNH04863 with other strains of species

| Strain of the present invention | NCBI strains | Species | NCBI strains as reference strains | | The strain of the present invention as reference strain | |
|---|---|---|---|---|---|---|
| | | | ANI | AF | ANI | AF |
| MNH048 63 | GCA_001652 705 | Christensen ella minuta DSM22607 | 83.532 | 65.00 % | 83.49 04 | 68.78 % |
| MNH048 63 | GCA_900087 015 | Christensen ella timonensis Marseille-P2437 | 78.87 73 | 21.02 % | 78.80 01 | 26.05 % |
| MNH048 63 | GCA_900155 415 | Christensen ella massiliensis Marseille-p2438 | 79.81 04 | 35.10 % | 79.83 44 | 42.79 % |

The mode strain with the highest genomic similarity was *Christensenella minuta,* with an average nucleotide identity (ANI) of 83.5% and a genome coverage of 68.78%. According to the criterion that ANI >95% defines the same species, the genome-relatedness analysis based on average nucleotide identity (ANI) in the present application demonstrated that strain MNH04863 was significantly different from other species within the *Christensenella sp. (C. minuta, C. timonensis, C. massiliensis),* and the ANI value with the closest related species, *Christensenella minuta,* was less than 83.5%. Several physiological, biochemical, and genotypic characteristics distinguish the strain of the present invention from related species.

The phylogenetic relationship between *Christensenella sp.* MNH04863 and other existing species within the *Christensenella* is shown in the phylogenetic tree in FIG. 4. The phylogenetic tree was constructed by using MEGA-X software. First, the 16S rRNA gene sequences of all strains within the *Christensenella sp.* were retrieved from the LPSN database. Then, multiple sequence alignment of the 16S sequences was performed using the MUSCLE method. Finally, a phylogenetic tree was constructed using the maximum likelihood method.

Example 3. Genomic Analysis of *Christensenella sp.* MNH04863

I. Analysis of potential virulence genes:

The analysis of potential virulence factors and related genes within the genome was performed by aligning against the Virulence Factor Database (VFDB, http://www.mge.ac.cn/cgi-bin/VFs/v5/main.cgi, last updated on September 19, 2019) using NCBI blastp (version 2.7.1+). The detailed alignment results are shown in Table 3.

Table 3: List of potential virulence genes in *Christensenella sp.* MNH04863.

| Strain gene | VFDB gene | Gene Name | Alignment consistency (%) |
|---|---|---|---|
| MNH04863_00269 | VFG037028 | katA | 63.655 |
| MNH04863_01151 | VFG000077 | clpP | 64.767 |
| MNH04863_01175 | VFG001855 | htpB | 60.985 |
| MNH04863_02839 | VFG002377 | ddhA | 60.618 |

II. Analysis of potential primary metabolic gene clusters:

The analysis of potential primary metabolic gene clusters within the genome was performed using gutSMASH (version 1.0.0). The detailed alignment results are shown in Table 4.

Table 4: List of potential primary metabolic gene clusters in *Christensenella sp.* MNH04863.

| Gene Cluster Range | Type | Fr om | T o | Most Similar Known Gene Clusters | Abbre viation | Simi larity |
|---|---|---|---|---|---|---|
| Region 1.1 | Others_HGD_unassigned | 95 930 | 120183 | | | |
| Region 1.2 | Flavoenzyme_AA_peptides_catabolismsuccinate2propionate | 42 1067 | 465963 | Glutamate to butyrate C. symbiosum | BUT | 30% |
| Region 1.3 | OD_fatty_acids | 47 8330 | 510607 | | | |
| Region 2.1 | TPP_AA_metabolismRnf_complex | 15 267 | 65752 | Rnf complex C. sporogenes | RNF | 100 % |
| Region 5.1 | Indoleacetate2scatole | 55 791 | 78550 | | | |
| Region 5.2 | Putrescine2spermidinePFOR_II_pathway | 16 6132 | 192521 | PFOR II pathway B. thetaiotaomicron | PFOR II | 100 % |
| Region 6.1 | Flavoenzyme_AA_peptides_catabolismFlavoenzyme_lipids_catabolism | 10 7989 | 1 41519 | | | |
| Region 7.1 | porAOD_fatty_acidsamin obutyrate2Butyrate | 21516 | 75090 | Aminobutyr ate to butyrate C. pasteurianum | AMIN OBUT | 40% |

III. Potential secondary metabolic gene clusters:

The analysis of potential secondary metabolic gene clusters within the genome was performed using antiSMASH 6 (version 6.0.1). The detailed alignment results are shown in Table 5.

Table 5: List of potential secondary metabolic gene clusters in *Christensenella sp.* MNH04863:

| Gene Cluster Range | Type | From | To | Most Similar Known Gene Clusters | Similarity |
|---|---|---|---|---|---|
| Region 4.1 | RiPP-like | 125988 | 136800 | - | - |
| Region 6.1 | ranthip eptide | 119183 | 140956 | - | - |

IV. Analysis of potential butyrate-producing genes:

The butyrate-producing capability of this strain was evaluated using genes related to the butyrate-producing pathway as a reference database. The genomic sequence of the strain was aligned against this reference database using NCBI blastp (version 2.7.1+). Detailed alignment results are shown in Table 6. The completeness of the butyrate-producing pathway was calculated, and it was found to be 42.86%.

Table 6: List of potential butyrate-producing genes in *Christensenella sp.* MNH04863:

| Strain Gene | Butyrate Pathway Name | Gene Name | Alignment Consistency (%) |
| --- | --- | --- | --- |
| MNH04863_02414 | 4aminobutyrate | AbfD-Isom | 82.365 |
| MNH04863_01249 | Glutarate | HgdB | 70.241 |
| MNH04863_02400 | Pyruvate | Bed | 81.794 |
| MNH04863_02398 | Pyruvate | EtfA | 74.926 |
| MNH04863_02399 | Pyruvate | EtfB | 79.231 |

V. Determination of Short-Chain Fatty Acids (SCFAs) of Strains

Determination of short-chain fatty acids (SCFA) produced by *Christensenella sp.* MNH04863

1. Preparation of bacterial cultures

The *Christensenella sp.* MNH04863 were inoculated in TSB liquid medium and anaerobically cultured at 37°C for 48 h. The bacterial cultures were collected by centrifugation and stored at -86°C of low temperature for subsequent use as samples.

2. Preparation of standard solutions

Standards substances of acetic acid, propionic acid, butyric acid, isobutyric acid, valeric acid, isovaleric acid, and hexanoic acid were weighed, and dissolved in ethyl acetate to prepare eight mixed standard concentration gradients: 0.1 $\mu$g/mL, 0.5 $\mu$g/mL, 1 $\mu$g/mL, 5 $\mu$g/mL, 10 $\mu$g/mL, 20 $\mu$g/mL, 50 $\mu$g/mL, and 100 $\mu$g/mL.
For each standard, 600 $\mu$L of the standard solution was taken, and 25 $\mu$L of 4-methylvaleric acid at a final concentration of 500 $\mu$M was added as an internal standard, mixed well and transferred into a sample vial for GC-MS detection. The injection volume was 1 $\mu$L with a split ratio of 10:1 under split injection mode.

3. Metabolite extraction

The sample was thawed on ice, and 80 mg of the sample was placed into a 2 mL glass centrifuge tube. 900 $\mu$L of 0.5% phosphoric acid was added to resuspend the sample, vortexed mixing for 2 min, and centrifuged at 14,000 g for 10 min. 800 $\mu$L of the supernatant was collected and extracted with an equal volume of ethyl acetate, vortexed mixing for 2 min, centrifuged at 14,000 g for 10 min. 600 $\mu$L of the upper organic phase was collected and added with 4-methylvaleric acid at a final concentration of 500 $\mu$M as an internal standard, mixed well and transferred into a sample vial for GC-MS detection. The injection volume was 1 $\mu$L with a split ratio of 10:1 under split injection mode.

4. Sample detection and analysis:

The samples were separated using a gas chromatography system equipped with Agilent DB-WAX capillary column (30 m $\times$ 0.25 mm ID $\times$ 0.25 $\mu$m). The temperature program was as follows: initial temperature of 90°C, increasing to 120°C at a rate of 10°C/min, then to 150°C at 5°C/min, and finally to 250°C at 25°C/min, maintained for 2 min. Helium was used as the carrier gas at a flow rate of 1.0 mL/min.
Mass spectrometric analysis was carried out using an Agilent 7890A/5975C GC-MS system.
Chromatographic peak areas and retention times were extracted by MSD ChemStation software. Calibration curves were plotted and the content of short chain fatty acids concentrations in the samples were calculated. The results are shown in Table 7.

Table 7: Results of short chain fatty acids (SCFA) production in *Christensenella sp.* MNH04863.

| SCFA yield (μg/g) | Aceti c acid | But yric acid | Isova leric acid | Valeric acid | He xanoic acid | Propa noic acid | Isobut yric acid |
|---|---|---|---|---|---|---|---|
| MNH04863 | 2694.8 | 360.3 | 0.845 | 0.19 | 4.18 | 5.60 | 1.14 |

As shown in the detection results, the *Christensenella sp.* MNH04863 produces high levels of acetic acid and butyric acid during growth. "High production" as described herein refers to a butyric acid yield of no less than 200 μg/g; preferably, the butyric acid yield is no less than 360 μg/g. The acetic acid yield is no less than 500 μg/g; preferably, the acetic acid yield is no less than 2694 μg/g. The detection results indicate that the actual butyric acid production level of the *Christensenella sp.* MNH04863 matches the prediction based on the integrity of the butyrate-producing pathway.

Example 4. Regulation of Immune Activity of Macrophages by *Christensenella sp.* MNH04863:

The experimental protocols are as follows:
Test strain: The MNH04863 strain from a glycerol stock was thawed at 37 °C and then inoculated onto anaerobic blood agar plates under anaerobic conditions for activation. The activated strain was subsequently inoculated into MM01 liquid medium, anaerobically cultured, and centrifuged. The bacterial pellet was resuspended in an appropriate amount of PBS. According to the instructions of the "Live/Dead BacLight Bacterial Viability Kits", flow cytometry counting of MNH04863 bacteria was performed to obtain the required strain sample for the experiment.
THP-1 cells (Wuhan Procell Life Science & Technology Co., Ltd.) were treated with a final concentration of 5 ng/mL PMA for 48 hr to differentiate them into M0 macrophages.
M0 macrophages were co-incubated with *Christensenella sp.* MNH04863 for 24 hr. *Christensenella sp.* MNH04863 was added at a MOI (ratio of viable bacteria count to cell count) = 10:1. M1 macrophage control group was also set up (M0 macrophages were induced with 20 ng/ml of IFNγ + 10 pg/ml of lipopolysaccharide, and LPS induction for 24 hr). After anaerobic incubation for 1 hr, a mixture of antibiotics (1 mg/mL of Ampicillin; 5 mg/mL of Streptomycin; and 1 mg/mL of Colistin) was added to kill the bacteria. The culture was continued for another 23 hr before collecting the supernatant. The antibiotics used are shown in Table 8.

Table 8: Antibiotics used in this example

| Antibiotics Types | Source | Final concentration |
|---|---|---|
| Ampicillin | Sigma-Aldrich | 1mg/ml |
| Streptomycin | MCE | 5mg/ml |
| Colistin | Sigma-Aldrich | 1mg/ml |

The concentrations of cytokines IL-8, IL-1β, MCP-1 and other factors in the supernatant were measured by flow cytometry using the BD™ Cytometric Bead Array (CBA) Human Soluble Protein Master Buffer Kit.
FIG. 5 shows a schematic diagram of the expression of various pro-inflammatory cytokines and chemokines induced in macrophages after co-culture of M0 macrophages and *Christensenella sp.* MNH04863 in this Example.
As shown in FIG. 5, the results indicate that after co-culturing M0 macrophages with *Christensenella sp.* MNH04863, macrophages clearly exhibited characteristics of differentiation towards M1-type macrophages. Among these, the levels of pro-inflammatory cytokines TNFα and IL-1β were significantly upregulated, and the levels of chemokines MCP-1 and IL-8 were also markedly increased.

Example 5. Regulation of Immune Activity of Primary PBMCs (Primary Peripheral Blood Mononuclear Cells) by Strain MNH04863:

I. This experimental example investigates the effect of MNH04863 on primary PBMCs.

Previous studies have shown that components of intestinal microbes have profound effects on the surrounding immune system and play a crucial role in the efficacy of tumor immunotherapy in cancer patients, constituting a key component of antitumor immunity and treatment effects. Intestinal microbes can interact with immune cells to jointly modulate the human immune system. Intestinal strains can induce differentiation of primary PBMCs into different cell types and secrete various inflammatory factors, thus they can be used for screening immune-modulating strains.

II. Experimental Methods:

Test Strain: The MNH04863 strain from a glycerol stock was thawed at 37 °C and then inoculated onto anaerobic blood agar plates within an anaerobic workstation for activation. The activated strain was subsequently inoculated into MM01 liquid medium, anaerobically cultured, and centrifuged. The bacterial pellet was resuspended in an appropriate amount of PBS. According to the instructions of the "Live/Dead BacLight Bacterial Viability Kits", flow cytometry counting of MNH04863 bacteria was performed to obtain the required strain sample for the experiment.

Commercially available primary PBMCs purchased from TPCS (batch number A19Z289100) were used, resuscitated, and cultured in PRMI1640 complete medium (10% of heat-inactivated FBS, containing 1% of L-glutamine, 0.1% of ps (penicillin-streptomycin mixture), and 10 mg/ml of DNase (DNase is used to avoid aggregation)).

Primary PBMCs were co-incubated with the strain for 24 hr. The strain was added at a MOI (ratio of viable bacteria count to cell count) = 1:1. PBS control group (PBS co-cultured with primary PBMCs for 24 hr) was also set up. After anaerobic incubation for 1 hr, a combination of antibiotics listed in the table below was added to kill the bacteria. The antibiotics used are shown in Table 9.

Table 9: List of antibiotics used in this example:

| Antibiotics Types | Source | Final concentration |
|---|---|---|
| Ampicillin | Sigma-Aldrich | 1mg/ml |
| Streptomycin | MCE | 5mg/ml |
| Colistin | Sigma-Aldrich | 1mg/ml |

After 24 h of co-incubation of strain MNH04863 with primary PBMC cells, the supernatant was collected. Using the BD™ Cytometric Bead Array (CBA) Human Soluble Protein Master Buffer Kit, cytokine concentrations in the supernatant were detected by flow cytometry.

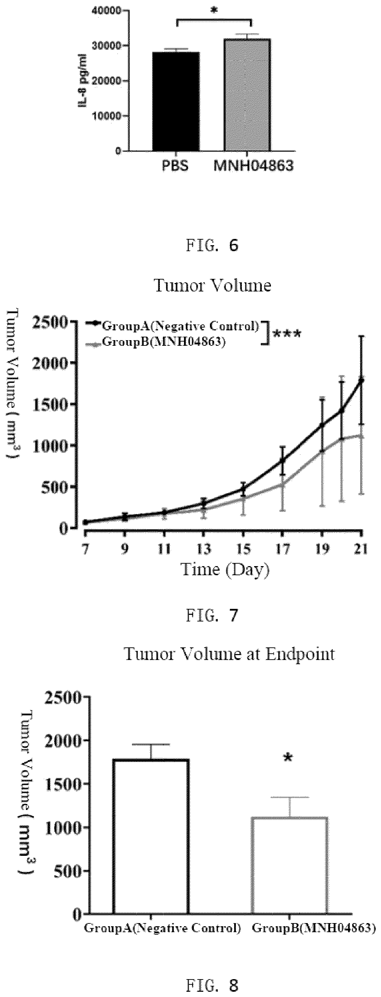

FIG. 6 shows a schematic diagram of the increase of chemokine expression induced by *Christensenella sp.* MNH04863 in this example in primary peripheral blood mononuclear cells.

As shown in FIG. 6, the results indicate that *Christensenella sp.* MNH04863 can induce a significant increase of the chemokine IL-8 in peripheral blood mononuclear cells.

Example 6. Verification of Therapeutic Effects of *Christensenella sp.* MNH04863 on Tumors of Lung Cancer:

This example verifies whether *Christensenella sp.* MNH04863 can be used for the tumor treatment of lung cancer using a mouse homologous tumor model to conduct experiments inhibiting lung cancer growth. This experimental protocol has passed ethical review by the Laboratory Animal Management and Use Committee of Moon Biotech.

1. Test strain: The *Christensenella sp.* MNH04863 strain from a glycerol stock was thawed at 37 °C and then inoculated onto anaerobic blood agar plates under anaerobic conditions for activation. The activated strain was subsequently inoculated into MM01 liquid medium for anaerobic cultivation to obtain sufficient quantities of culture. The cultured bacterial solution was centrifugated and concentrated, and the bacterial pellet was resuspended in solvent to obtain the subjects with the purity and the number of viable bacteria ($1 \times 10^9$ CFU/mL~$2 \times 10^{10}$ CFU/mL) that satisfy the requirements of the animal experiments.

2. Tumor cells: LLC1 mouse lung cancer cells; Source: Wuhan Procell Life Science & Technology Co., Ltd., Catalog No.: CL-0140.

3. Experimental animals: The experimental mice were C57BL/6J mice, aged 5-7 weeks, and purchased from Guangdong GemPharmatech Co., Ltd.

4. Animal experiment: Under normal feeding conditions, after the quarantine, subcutaneous injection of LLC1 lung cancer cells was performed to form an ectopic homologous tumor model. The inoculation volume was $2 \times 10^6$ cells/mouse, 0.1~0.3 mL/mouse. When the average tumor volume reached 80-100 mm$^3$, animals were randomly divided into two groups based on tumor volume:

Group A (Control); and
Group B (*Christensenella sp.* MNH04863).
Oral gavage administration began on the day of grouping, with the control group administering solvent and Group B administering *Christensenella sp.* MNH04863.

The gavage volume was 0.15~0.25 mL/mouse.

General observations were conducted once daily after administration during the quarantine and administering periods. Body weights of animals were recorded upon receipt of the animals, at the end of the quarantine, at each time of measuring tumor diameters after tumor cell inoculation, and before dissection at the experimental endpoint. The day of tumor cell inoculation was designated as Day 1 (D1). From D2 until the grouping, tumor diameters were measured once daily and tumor growth was recorded. After grouping, tumor diameters were measured every two days and tumor growth was recorded. When the average tumor volume reached $\geq 1000$ mm$^3$ (D19), tumor diameters were measured once daily and tumor growth was recorded. At the experimental endpoint, tumor diameters were measured and tumor growth was recorded before dissection.

The experimental endpoint was as follows: the experiment could be terminated if the average tumor volume in any group reached $\geq 1000$ mm$^3$, and the tumor volume in Group B was significantly smaller than that in Group A, or if the average tumor growth inhibition rate (TGI) in that group was $\geq 20\%$ compared to Group A; or the average tumor volume in any group exceeded 2000 mm$^3$. General clinical observations, body weight monitoring, and tumor volume (mm$^3$) measurements were conducted throughout the experiment. At the experimental endpoint, all surviving mice were dissected for tumor tissue collection, and statistical analyses such as calculating the tumor volume and endpoint tumor growth inhibition rate (TGI at endpoint) were conducted.

5. Experimental results of *Christensenella sp.* MNH04863 on tumor treatment of lung cancer

Table 10: Summary statistics of overall mouse conditions at experimental endpoint:

| Grou p | Num ber of Mice Enrolled | Num ber of mice at Experimen tal endpoint | Num ber of Euthanize d mice | Avera ge Weight Before Dissection (g) | Aver age Anatomica l Tumor Volume (mm$^3$) | Experime ntal Endpoint TGI (%) | Respo nse Rate (%) |
|---|---|---|---|---|---|---|---|
| Cont rol group | 10 | 10 | 0 | 23.91 | 1788. 30 | 0.00% | 10% |
| Grou p B | 10 | 10 | 0 | 23.46 | 1121. 09 | 38.86% | 60% |

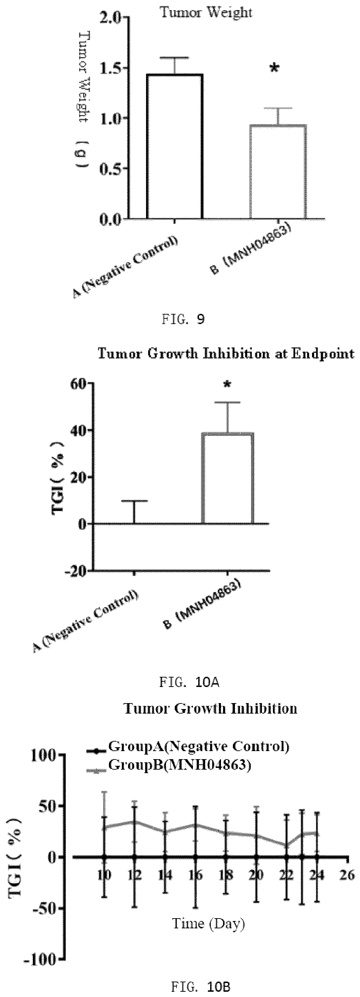

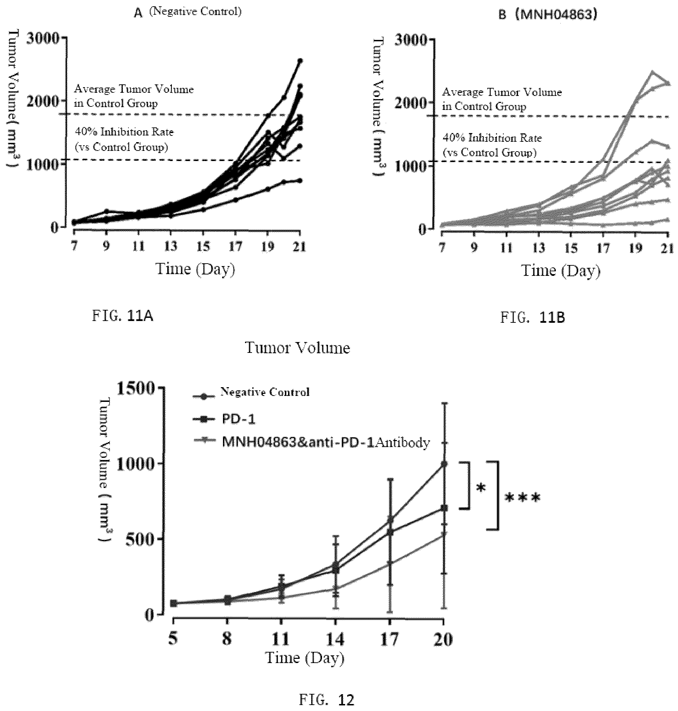

At the experimental endpoint (D21), surviving mice were euthanized by cervical dislocation. The results are summarized in Table 10. At the endpoint, the average body weight before dissection in the control group was 23.91 g, and the average tumor volume of mice in the control group was 1788.30 mm$^3$. In the *Christensenella sp.* MNH04863-treated group (Group B), the average tumor volume of mice was 1121.09 mm$^3$, and the average body weight before dissection was 23.46 g. The average tumor volume of mice in the *Christensenella sp.* MNH04863-treated group was clearly lower than that in the control group. At the endpoint, the average tumor growth inhibition rate (TGI) of mice in the *Christensenella sp.* MNH04863-treated group was 38.86%, indicating a significant inhibitory effect on tumor growth. Additionally, as shown in FIG. 7, the statistical analysis results of tumor volume curves in Group B *(Christensenella sp.* MNH04863) during the experiment were significantly lower than those of Group A (control group). As shown in FIG. 8, the statistical analysis results of final tumor volumes of mice at the experimental endpoint in Group B *(Christensenella sp.* MNH04863) during the experiment were significantly lower than those of Group A (control group). As shown in FIG. 9, the statistical analysis results of final tumor weights of mice at the experimental endpoint in Group B *(Christensenella sp.* MNH04863) during the experiment were significantly lower than those of Group A (control group). As shown in FIGs. 10A and 10B, the statistical analysis results of final tumor growth inhibition rates of mice at the experimental endpoint in Group B *(Christensenella sp.* MNH04863) during the experiment were significantly higher than those of Group A (control group). As shown in FIGs. 11A and 11B, at the experimental endpoint, the response rate in Group A was 10%, while the response rate in Group B *(Christensenella sp.* MNH04863) was 60%, demonstrating that *Christensenella sp.* MNH04863 significantly increased the response rate to tumor treatment.

Example 7. Verification Experiment of Therapeutic Effects of *Christensenella sp.* MNH04863 in Combination with Anti-PD-1 Antibody on Tumors of Lung Cancer:

I. Experiment Methods:

To verify the therapeutic effects of *Christensenella sp.* MNH04863 in combination with anti-PD-1 antibody, a mouse homologous tumor model was used to conduct experiments inhibiting lung cancer growth. This experimental protocol has passed ethical review by the Laboratory Animal Management and Use Committee of Moon Biotech.

Test strain: The *Christensenella sp.* MNH04863 strain from a glycerol stock was thawed at 37 °C and then inoculated onto anaerobic blood agar plates under anaerobic conditions for activation. The activated strain was subsequently inoculated into MM01 liquid medium for anaerobic cultivation to obtain sufficient quantities of culture. The cultured bacterial solution was centrifuged and concentrated, and the bacterial pellet was resuspended in solvent to obtain the subjects with the purity and the number of viable bacteria ($1\times10^9$ CFU/mL~$2\times10^{10}$ CFU/mL) that satisfy the requirements of the animal experiments.

Anti-PD-1 antibody: anti-PD-1 antibody (RMP1-14), prepared as a 2 mg/mL solution in saline; Source: BioXcell.

Tumor cells: LLC1 mouse lung cancer cells; Source: Wuhan Procell Life Science & Technology Co., Ltd., Catalog No.: CL-0140.

Experimental animals: The experimental mice were C57BL/6J mice, aged 5-7 weeks, and purchased from Guangdong GemPharmatech Co., Ltd.

Animal experiment: Under normal feeding conditions, after the quarantine, subcutaneous injection of LLC1 lung cancer cells was performed to form an ectopic homologous tumor model. The inoculation volume was 0.1~0.3 mL/mouse. When the average tumor volume reached 60-100 mm$^3$, animals were randomly divided into 10 groups based on tumor volume:

Group A (negative control);
Group B (positive control: anti-PD-1 antibody);
Group D *(Christensenella sp.* MNH04863 & anti-PD-1 antibody); and
Group C as well as Groups E-J are other test substances.

Oral gavage administration began on the day of grouping. Group A was administered with solvent and saline, Group B was administered with solvent and anti-PD-1 antibody, and Group D was administered with *Christensenella sp.* MNH04863 and anti-PD-1 antibody. Solvent and *Christensenella sp.* MNH04863 were administered via oral gavage at a volume of 0.1~0.3 mL/mouse; anti-PD-1 antibody and saline were administered via intraperitoneal injection at a dose of 5~15 mg/kg every 3 days for a total of 5 doses. Body weights of animals were recorded upon receipt of the animals and at the end of the quarantine. After tumor cell inoculation, body weights of animals were measured twice weekly, including on days when intraperitoneal injections were administered. The day of tumor cell inoculation was designated as Day 1 (D1). From D5 until the grouping, tumor diameters were measured once daily; after grouping, tumor diameters were measured every three days. When the average tumor volume reached $\geq$1000 mm$^3$, tumor diameters were measured once daily and tumor growth was recorded.

The experiment could be terminated if the average tumor volume in any group reached $\geq$1000 mm$^3$, and the tumor volume in any of Groups C to J was significantly smaller than that in Groups A and B, or if the average tumor growth inhibition rate (TGI) in that group was $\geq$20% compared to Group B. Alternatively, the experiment could be terminated if the average tumor volume in any group exceeded 2000 mm$^3$. General clinical observations, body weight monitoring, and tumor volume (mm$^3$) measurements were conducted throughout the experiment. At the experimental endpoint, all surviving mice were dissected for tumor tissue collection, the tumor weights were measured and the tumor volumes were calculated. Finally, statistical analyses and comparisons were performed on the tumor volume curve changes, endpoint tumor volume, endpoint tumor weight, and endpoint tumor growth inhibition rate (TGI) *etc.*

II. Experimental results of *Christensenella sp.* MNH04863 in combination with anti-PD-1 antibody on tumor treatment of lung cancer:

At the experimental endpoint (D20), the surviving mice were dissected and their tumor tissues were weighed. The results are summarized in Table 11.

Table 11: Summary statistics of overall mouse conditions at experimental endpoint:

| Gr oup | Nu mber of Mice Enrolled | Nu mber of mice at Experime ntal endpoint | Nu mber of Euthaniz ed mice | Ave rage Anatomi cal Tumor Weight (g) | Ave rage Weight Before Dissectio n (g) | Ave rage Tumor Volume at Endpoint (m m$^3$) | Experi mental Endpoint TGI (%) | Resp onse Rate (%) |
|---|---|---|---|---|---|---|---|---|
| Gr oup A (Negtiv e Control ) | 8 | 8 | 0 | 1.02 | 23.8 6 | 100 7.22 | 0 | 25% |

(continued)

| Gr oup | Nu mber of Mice Enrolled | Nu mber of mice at Experime ntal endpoint | Nu mber of Euthaniz ed mice | Ave rage Anatomi cal Tumor Weight (g) | Ave rage Weight Before Dissectio n (g) | Ave rage Tumor Volume at Endpoint (m m$^3$) | Experi mental Endpoint TGI (%) | Resp onse Rate (%) |
|---|---|---|---|---|---|---|---|---|
| Gr oup B (Positiv e Control ) | 8 | 8 | 0 | 0.84 | 24.4 3 | 715 | 31.4 | 25% |
| Gr oup D (MN-O-863& PD-1 anti-bod y) | 8 | 8 | 0 | 0.56 | 24.4 1 | 534. 27 | 50.8 | 62.5 % |

FIG. 12 shows a graph of the tumor volume of mice in the tumor verification experiment of lung cancer in this example. As shown in FIG. 12, during the experiment, the statistical analysis results of the tumor volume curve in Group D *(Christensenella sp.* MNH04863 & PD-1) are significantly lower than those in Group A (negative control).

As shown in Table 11, at the experimental endpoint, the average tumor volume of mice in the negative control group was 1007.22 mm$^3$, and the average tumor tissue weight was approximately 1.02 g. In the positive control group, the average tumor volume of mice was 715.00 mm$^3$, and the average tumor tissue weight was approximately 0.84 g. In the *Christensenella sp.* MNH04863 & anti-PD-1 antibody-treated group, the average tumor volume of mice was 534.27 mm$^3$, and the average tumor weight was approximately 0.56 g. These results demonstrate that both tumor volume and tumor weight in the *Christensenella sp.* MNH04863 & anti-PD-1 antibody-treated group are markedly reduced compared to the control group.

As shown in Table 11, at the experimental endpoint, the tumor growth inhibition rate (TGI) in Group B (positive control) was 31.4%, while in Group D *(Christensenella sp.* MNH04863 & PD-1), the TGI was 50.8%, which is significantly higher than that in the positive control group.

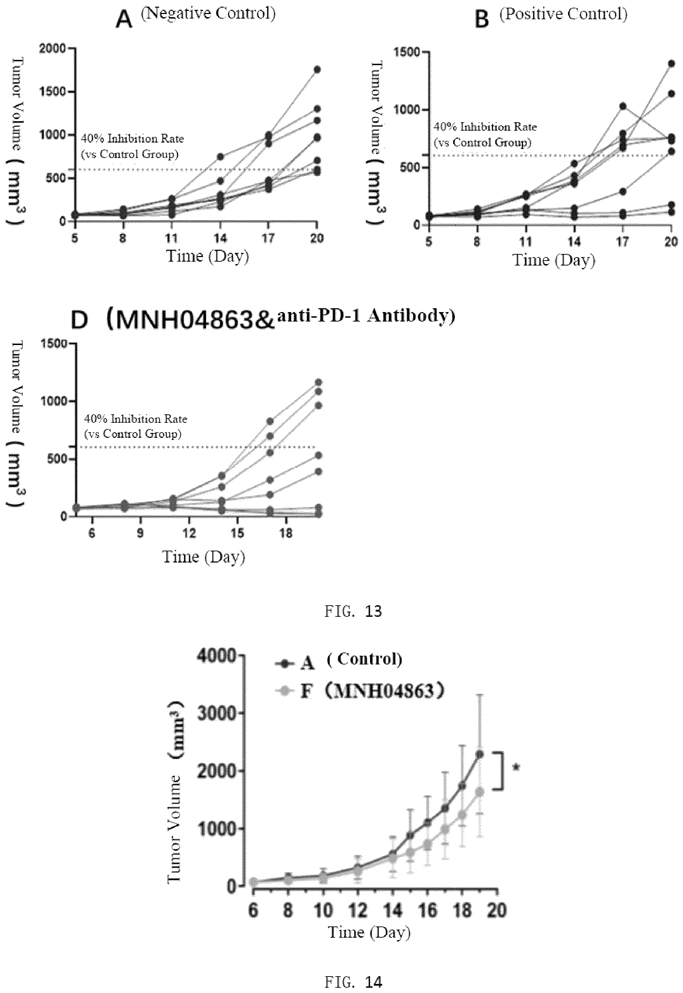

As shown in FIG. 13, at the experimental endpoint, the response rate in Group A (negative control) was 25.0%, and the response rate in Group B (positive control) was also 25.0%. This indicates that lung cancer is a refractory tumor, and treatment with anti-PD-1 antibody did not effectively improve the response rate compared to the negative control. However, the combination of *Christensenella sp.* MNH04863 of the present application with anti-PD-1 antibody significantly increased the response rate to 62.5% (see response rate in Group D: *Christensenella sp.* MNH04863 & PD-1). The combination of *Christensenella sp.* MNH04863 with anti-PD-1 antibody clearly enhanced the therapeutic response rate, confirming that *Christensenella sp.* MNH04863 can synergize with anti-PD-1 antibody to improve therapeutic efficacy. These findings suggest that *Christensenella sp.* MNH04863 of the present application can be used in the treatment of clinically refractory tumors.

Example 8. Verification Experiment of Therapeutic Effects of *Christensenella sp.* MNH04863 on Tumors of Liver Cancer:

This example verifies whether *Christensenella sp.* MNH04863 can be used for the tumor treatment of liver cancer using a mouse homologous tumor model to conduct experiments inhibiting liver cancer growth. This experimental protocol has passed ethical review by the Laboratory Animal Management and Use Committee of Moon Biotech.

1. Test strain: The *Christensenella sp.* MNH04863 strain from a glycerol stock was thawed at 37 °C and then inoculated onto anaerobic blood agar plates under anaerobic conditions for activation. The activated strain was subsequently inoculated into MM01 liquid medium for anaerobic cultivation to obtain sufficient viable bacteria. The cultured bacterial solution was centrifuged and concentrated, and the bacterial pellet was resuspended in solvent to obtain the subjects with the purity and the number of viable bacteria ($1\times10^9$ CFU/mL $\sim 2.0\times10^{10}$ CFU/mL) that satisfy the requirements of the animal experiments.

2. Tumor cells: H22 mouse hepatocellular carcinoma cells; Source: Nanjing Cobioer Biotechnology Co., Ltd., Catalog No.: CBP60230.

3. Experimental animals: The experimental mice were BALB/C mice, aged 5-6 weeks, and purchased from Guangdong Sijia Jingda Biotechnology Co., Ltd.

4. Animal experiment: Under normal feeding conditions, after the quarantine, subcutaneous injection of H22 liver cancer cells was performed to form an ectopic homologous tumor model. The inoculation volume was $2\times10^5$ cells/mouse, 0.1 mL/mouse.

When the average tumor volume reached 80-100 mm$^3$, animals were randomly divided into six groups based on tumor volume:

Group A (Control); and
Group F (Christensenella sp. MNH04863).

Oral gavage administration began on the day of grouping, with the control group administering solvent and Group F administering Christensenella sp. MNH04863 at a gavage volume of 0.2 mL/mouse, with a dosing frequency of once daily.

General observations were conducted once daily after administration during the quarantine and administering periods. Body weights of animals were recorded upon receipt of the animals, at the end of the quarantine, at each time of measuring tumor diameters after tumor cell inoculation, and before dissection at the experimental endpoint. The day of tumor cell inoculation was designated as Day 1. From Day 2 until the grouping, tumor diameters were measured once daily and tumor growth was recorded.

When the average tumor volume reached 80-100 mm$^3$, animals were divided into groups. After grouping, tumor diameters were measured every two days and tumor growth was recorded. When the average tumor volume reached ≥500 mm$^3$ (D15), tumor diameters were measured once daily and tumor growth was recorded. At the experimental endpoint, tumor diameters were measured and tumor growth was recorded before dissection.

The experimental endpoint was as follows: the experiment could be terminated if the average tumor volume in any group reached ≥1000 mm$^3$, and the tumor volume in Group B was significantly smaller than that in Group A, or if the average tumor growth inhibition rate (TGI) in that group was ≥20% compared to Group A. Alternatively, the experiment would be terminated if the average tumor volume in any group exceeded 2000 mm$^3$. General clinical observations, body weight monitoring, and tumor volume (mm$^3$) measurements were conducted throughout the experiment. At the experimental endpoint, all surviving mice were dissected for tumor tissue collection, tumor weights were measured, and tumor volumes were calculated.

5. Experimental Analysis:

Statistical analyses and comparisons were performed on above experimental data including tumor volume curve changes, endpoint tumor volume, endpoint tumor weight, and endpoint tumor growth inhibition rate (TGI) etc.

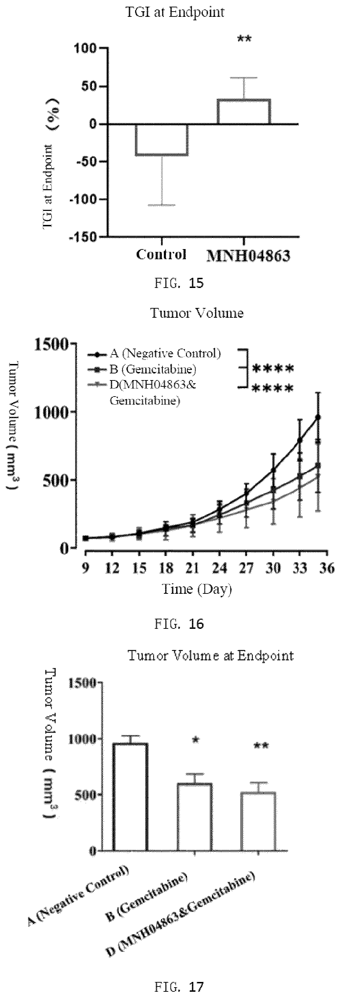

As shown in FIG. 14, during the experiment, the statistical analysis of the tumor volume curve in Group F (Christensenella sp. MNH04863) was significantly lower than that of Group A (control group). At the experimental endpoint (D19), surviving mice were dissected and their tumor tissues were weighed. The results are summarized in Table 12. At the experimental endpoint, the average tumor volume of mice in the control group was 2292.35 mm$^3$, while the average tumor volume of mice in the Christensenella sp. MNH04863-treated group was 1636.01 mm$^3$. The average tumor tissue weight in the control group was approximately 3.44 g, while the average tumor tissue weight in the Christensenella sp. MNH04863-treated group was approximately 2.99 g. Both tumor volume and tumor weight in the Christensenella sp. MNH04863-treated group were significantly lower than that in the control group (Table 12). As shown in FIG. 15, at the experimental endpoint, the tumor growth inhibition rate (TGI) in Group F (Christensenella sp. MNH04863) was significantly higher than that in Group A (control).

Table 12. Summary statistics of overall mouse conditions at experimental endpoint:

| Group | Number of Mice Enrolled | Number of mice at Experimental endpoint | Number of Euthanized mice | Average Anatomical Tumor Weight (g) | Average Weight Before Dissection (g) | Average Tumor Volume at Endpoint (m m$^3$) | Experimental Endpoint TGI (%) |
|---|---|---|---|---|---|---|---|
| Group A (Control) | 1 6 | 11 | 5 | 3.44 | 32.19 | 229 2.35 | 0 |
| Group F (Christensenella sp. MNH04863) | 1 6 | 10 | 6 | 2.99 | 32.13 | 163 6.01 | 32.94 % |

Example 9. Verification Experiment of Therapeutic Effects of *Christensenella sp.* MNH04863 in Combination with Anti-PD-1 Antibody on Tumors of Liver Cancer:

I. Experiment Methods:

To verify the therapeutic effects of *Christensenella sp.* MNH04863 in combination with anti-PD-1 antibody, a mouse homologous tumor model was used to conduct experiments inhibiting liver cancer growth. This experimental protocol has passed ethical review by the Laboratory Animal Management and Use Committee of Moon Biotech.

Test strain: The *Christensenella sp.* MNH04863 strain from a glycerol stock was thawed at 37°C and then inoculated onto anaerobic blood agar plates under anaerobic conditions for activation. The activated strain was subsequently inoculated into MM01 liquid medium for anaerobic cultivation to obtain sufficient viable bacteria. The cultured bacterial solution was centrifuged and concentrated, and the bacterial pellet was resuspended in solvent obtain the subjects with the purity and the number of viable bacteria ($1\times10^9$ CFU/mL~$2\times10^{10}$CFU/mL) that satisfy the requirements of the animal experiments.

Anti-PD-1 Antibody: Anti-PD-1 antibody (RMP1-14), prepared as a 2 mg/mL solution in saline; Source: BioXcell.

Tumor Cells: H22 mouse hepatocellular carcinoma cells; Source: Nanjing Cobioer Biosciences Co., Ltd., Catalog No.: CBP60230.

Experimental Animals: The experimental mice were C57BL/6J mice, aged 5-7 weeks, and purchased from Guangdong GemPharmatech Co., Ltd.

Animal Experiment: Under normal feeding conditions, after the quarantine, subcutaneous injection of H22 mouse hepatocellular carcinoma cells was performed to form an ectopic homologous tumor model. The inoculation volume was 0.1~0.3 mL/mouse. When the average tumor volume reached 60-100 mm$^3$, animals were randomly divided into 10 groups based on tumor volume:

Group A (negative control);
Group B (positive control: anti-PD-1 antibody);
Group D *(Christensenella sp.* MNH04863 & anti-PD-1 antibody); and
Group C and E-J are other test substances.

Oral gavage administration began on the day of grouping. Group A was administered with solvent and saline, Group B was administered with solvent and anti-PD-1 antibody, and Group D was administered with *Christensenella sp.* MNH04863 and anti-PD-1 antibody. Solvent and *Christensenella sp.* MNH04863 were administered via oral gavage at a volume of 0.1~0.3 mL/mouse; anti-PD-1 antibody and saline were administered via intraperitoneal injection at a dose of 3 mg/kg every 3 days for a total of 5 doses. Body weights of animals were recorded upon receipt of the animals and at the end of the quarantine. After tumor cell inoculation, body weights of animals were measured twice weekly, including on days when intraperitoneal injections were administered. The day of tumor cell inoculation was designated as D1. From D5 until the grouping, tumor diameters were measured once daily; after grouping, tumor diameters were measured every three days. When the average tumor volume reached ≥1000 mm$^3$, tumor diameters were measured once daily and tumor growth was recorded.

The experiment could be terminated if the average tumor volume in any group reached ≥1000 mm$^3$, and the tumor volume in any of Groups C to J was significantly smaller than that in Groups A and B, or if the average tumor growth inhibition rate (TGI) in that group was ≥20% compared to Group B. Alternatively, the experiment could be terminated if the average tumor volume in any group exceeded 2000 mm$^3$. General clinical observations, body weight monitoring, and tumor volume (mm$^3$) measurements were conducted throughout the experiment. At the experimental endpoint, all surviving mice were dissected for tumor tissue collection, the tumor weights were measured and the tumor volumes were calculated. Finally, statistical analyses and comparisons were performed on the tumor volume curve changes, endpoint tumor volume, endpoint tumor weight, and endpoint tumor growth inhibition rate (TGI) *etc.*

II. Experimental results of *Christensenella sp.* MNH04863 in combination with Anti-PD-1 antibody on tumor treatment of liver cancer:

At the experimental endpoint (D20), the surviving mice were dissected and their tumor tissues were weighed. The results are summarized in Table 13.

Table 13: Experimental results of *Christensenella sp.* MNH04863 in combination with anti-PD-1 antibody on tumor treatment of liver cancer:

| Groups | Mice Enrolled Numbe r | Mice at experimental endpoint Number | Euthaniz ed mice Number | Average Weight Before Dissection (g) | Average Tumor Volume at Endpoint (mm$^3$) | Endpoin t tumor suppression rate (%) |
|---|---|---|---|---|---|---|
| Group A | 8 | 4 | 4 | 27.06 | 2241.39 | 0 |
| Negative control | | | | | | |
| Group B (Positive control: anti-PD-1 antibody); | 8 | 8 | 0 | 27.73 | 936.11 | 60.6 |
| Group D (*Christense nella sp.* MNH04863 & anti-PD-1 antibody) | 8 | 8 | 0 | 27.61 | 740.19 | 69.79 |

At the experimental endpoint, the tumor growth inhibition rate (TGI) in Group B (positive control) was 60.64%, while in Group D (*Christensenella sp.* MNH04863 & anti-PD-1 antibody), the TGI was 69.79%, which is significantly higher than that in the positive control group. Additionally, the combination of *Christensenella sp.* MNH04863 with anti-PD-1 antibody markedly improved the response rate to treatment.

Example 10. Verification Experiment of Therapeutic Effects of Different Formulations of *Christensenella sp.* MNH04863 on Tumors of Liver Cancer:

This example verifies whether *Christensenella sp.* MNH04863 and the bacterial strain-based API thereof can be used for tumor treatment of liver cancer using a mouse homologous tumor model to conduct experiments inhibiting liver cancer growth. This experimental protocol has passed ethical review by the Laboratory Animal Management and Use Committee of Moon Biotech.

1. Test Strain: The MNH04863 strain from a glycerol stock was thawed at 37°C and then inoculated onto anaerobic blood agar plates under anaerobic conditions for activation. The activated strain was subsequently inoculated into MM01 liquid medium for anaerobic cultivation to obtain sufficient viable bacteria. The cultured bacterial solution was centrifuged and concentrated, and the bacterial pellet was resuspended in solvent to obtain the subjects with the purity and the number of viable bacteria ($1\times10^9$ CFU/mL~$2\times10^{10}$ CFU/mL) that satisfy the requirements of the animal experiments.

2. Tumor Cells: H22 mouse hepatocellular carcinoma cells; Source: Nanjing Cobioer Biosciences Co., Ltd., Catalog No.: CBP60230.

3. Experimental Animals: The experimental mice were BALB/c mice, aged 5-6 weeks, and purchased from Guangdong Sijia Jingda Biotechnology Co., Ltd.

4. Animal Experiment: Under normal feeding conditions, after the quarantine, subcutaneous injection of H22 liver cancer cells was performed to form an ectopic homologous tumor model. The inoculation volume was $2\times10^5$ cells/mouse, 0.1~0.3 mL/mouse. When the average tumor volume reached 60-100 mm$^3$, animals were randomly divided into 5 groups based on tumor volume:

Group A (Control)
Group B (*Christensenella sp.* MNH04863 Soft Capsules)
Group C (*Christensenella sp.* MNH04863 Enteric-Coated Capsules)
Group D (*Christensenella sp.* MNH04863 Electrostatic Spray)
Group E (*Christensenella sp.* MNH04863 Live Bacterial Suspension)

Oral gavage administration began on the day of grouping. Control Group was administered with solvent, and Groups B-E were administered with *Christensenella sp.* MNH04863 soft capsules API, *Christensenella sp.* MNH04863 enteric-coated capsules API, *Christensenella sp.* MNH04863 electrostatic spray API, and *Christensenella sp.* MNH04863 live bacterial suspension, respectively.

The gavage volume was 0.15~0.25 mL/mouse/dose, with a dosing frequency of once daily.

General observations were conducted once daily after administration during the quarantine and administering periods. Body weights of animals were recorded upon receipt of the animals, at the end of the quarantine, at each time of measuring tumor diameters after tumor cell inoculation, and before dissection at the experimental endpoint. The day of tumor cell inoculation was designated as Day 1 (D1). From D2 until the grouping, tumor diameters were measured once daily and tumor growth was recorded. After grouping, tumor diameters were measured every two days and tumor growth was recorded. When the average tumor volume reached $\geq 1000$ mm$^3$ (D23), tumor diameters were measured once daily and tumor growth was recorded. At the experimental endpoint, tumor diameters were measured and tumor growth was recorded before dissection.

The experimental endpoint was as follows: the experiment could be terminated if the average tumor volume in any group reached $\geq 1000$ mm$^3$, and the tumor volume in any of Groups B-E was significantly smaller than that in Group A, or if the average tumor growth inhibition rate (TGI) in that group was $\geq 20\%$ compared to Group A; or if the average tumor volume in any group exceeded 2000 mm$^3$. General clinical observations, body weight monitoring, and tumor volume (mm$^3$) measurements were conducted throughout the experiment. At the experimental endpoint, all surviving mice were dissected for tumor tissue collection, and statistical analyses such as calculating the tumor volume and endpoint tumor growth inhibition rate (TGI at endpoint) were conducted.

Table 14: Experimental results of different formulations of *Christensenella sp.* MNH04863 on tumors of liver cancer:

| Group | Number of Mice Enrolled | Number of mice at Experimental endpoint | Number of Euthanized mice | Average Anatomical Tumor Weight (g) | Average Weight Before Dissection (g) | Average Tumor Volume at Endpoint (mm$^3$) | Experimental Endpoint TGI (%) | Response Rate (%) |
|---|---|---|---|---|---|---|---|---|
| PBS-Cys | 10 | 9 | 1 | 0.97 | 28.33 | 1101.49 | 0 | 11.1 % |
| MNO-863 Soft Capsules | 10 | 9 | 1 | / | 28.37 | 803.80 | 29.7 | 40% |
| MNO-863 Enteric-coated Capsules | 10 | 8 | 2 | / | 28.40 | 883.19 | 21.9 | 25% |
| MNO-863 Electrostatic Spray | 10 | 10 | 0 | 0.74 | 28.66 | 742.37 | 35.6 | 30% |
| MNO-863 Live Bacterial Suspension | 10 | 9 | 1 | 0.77 | 29.12 | 793.46 | 30.7 | 22.22% |

At the experimental endpoint, the response rate of Group A (negative control) was 11.1%, and the response rates of Groups B, C, D, and E were 40%, 25%, 30%, and 22.22%, respectively. Therefore, *Christensenella sp.* MNH04863 can be formulated into various dosage forms (such as different types of capsules (e.g., enteric-coated capsules or soft capsules), electrostatic spray formulations, or bacterial suspensions), all of which demonstrate effective antitumor therapeutic effects.

Example 11. Verification Experiment of the Therapeutic Effects of *Christensenella sp.* MNH04863 in Combination with Gemcitabine on Tumor of Pancreatic Cancer:

I. Experiment Methods:

To verify the therapeutic effects of *Christensenella sp.* MNH04863 in combination with gemcitabine on tumor treatment, a mouse homologous tumor model was used to conduct experiments inhibiting pancreatic cancer growth. This experimental protocol has passed ethical review by the Laboratory Animal Management and Use Committee of Moon Biotech.

Gemcitabine is a pyrimidine-based antimetabolite drug and belongs to the class of cell cycle-specific agents. It primarily acts during the DNA synthesis phase, and under certain conditions, it can also block the transition of cells from the pre-synthesis phase to the synthesis phase. Once inside tumor cells, gemcitabine is converted into gemcitabine triphosphate with activity, which interferes with DNA synthesis and repair processes. This leads to the inhibition of tumor cell proliferation and induction of tumor cell apoptosis. Gemcitabine was first approved by the FDA in the United States in 1996 for the treatment of pancreatic cancer, and subsequently in 1998 for the treatment of non-small cell lung cancer. With characteristics including a broad anticancer spectrum, a unique mechanism of action, low toxicity, no cross-resistance with other chemotherapeutic agents, and no additive toxic effects, gemcitabine is now approved for use in more than 90 countries worldwide. It has become a first-line treatment for non-small cell lung cancer and the "gold standard" for the treatment of pancreatic cancer.

Test Strain: The *Christensenella sp.* MNH04863 strain from a glycerol stock was thawed at 37°C and then inoculated onto anaerobic blood agar plates under anaerobic conditions for activation. The activated strain was subsequently inoculated into MM01 liquid medium for anaerobic cultivation to obtain sufficient viable bacteria. The cultured bacterial solution was centrifuged and concentrated, and the bacterial pellet was resuspended in solvent to obtain the subjects with the purity and the number of viable bacteria ($1\times10^9$ CFU/mL~$2\times10^{10}$CFU/mL) that satisfy the requirements of the animal experiments.

Tumor cells: KPC mouse pancreatic cancer cells; Source: Fubio (Suzhou) Biomedical Technology Co., Ltd., Catalog No.: FNC0071.

Experimental animals: The experimental mice were C57BL/6J mice, aged 7-8 weeks, and purchased from Guangdong GemPharmatech Co., Ltd.

Animal experiment: Under normal feeding conditions, after the quarantine, subcutaneous injection of KPC pancreatic cancer cells was performed to form an ectopic homologous tumor model. The inoculation volume was $3\times10^6$ cells/mouse, 0.1~0.3 mL/mouse. When the average tumor volume reached 60-100 mm$^3$, animals were randomly divided into 10 groups based on tumor volume:

Group A (Negative Control);
Group B (Positive Control: Gemcitabine);
Group D (*Christensenella sp.* MNH04863 & Gemcitabine); and
Groups C and E-J are other test substances.

Oral gavage administration began on the day of grouping. Group A was administered with solvent and saline; Group B was administered with solvent and gemcitabine; and Group D was administered with *Christensenella sp.* MNH04863 and gemcitabine. Solvent and *Christensenella sp.* MNH04863 were administered via oral gavage at a volume of 0.1~0.3 mL/mouse. When the average tumor volume in the negative control Group A reached 120-150 mm$^3$ or 7 days after gavage administration, gemcitabine and saline were administered via intraperitoneal injection at a dose of 5~15 mg/kg every 3 days for a total of 5 doses. Body weights of animals were recorded upon receipt of the animals and at the end of the quarantine. After tumor inoculation, body weights of animals were measured twice weekly, including on days when intraperitoneal injections were administered. The day of tumor cell inoculation was designated as D1. From D5 until the grouping, tumor diameters were measured once daily. After grouping, tumor diameters were measured every 3 days. When the average tumor volume reached ≥600 mm$^3$, tumor diameters were measured once daily, and tumor growth was recorded.

The experiment could be terminated if the average tumor volume in any group reached ≥ 600 mm$^3$, and the tumor volume in any of Groups C to J was significantly smaller than that in Group A and Group B, or the average tumor growth inhibition rate in that group (TGI) was ≥20% higher than that of Group B. Alternatively, the experiment could be terminated if the average tumor volume in any group exceeded 2000 mm$^3$. General clinical observations, body weight monitoring, and tumor volume (mm$^3$) measurements were conducted throughout the experiment. At the experimental endpoint, all surviving mice were dissected for tumor tissue collection. Tumor weight was measured and tumor volume was calculated. Finally, statistical analyses and comparisons were performed on the tumor volume curve changes, endpoint tumor volume, endpoint tumor weight, and endpoint tumor growth inhibition rate (TGI) *etc.*

II. Experimental results of *Christensenella sp.* MNH04863 in combination with Gemcitabine on tumor treatment of pancreatic cancer:

Table 15: Experimental results of *Christensenella sp.* MNH04863 in Combination with Gemcitabine on tumor treatment of Pancreatic Cancer:

| Group | Num ber of Mice Enrolled | Num ber of mice at Experimen tal endpoint | Num ber of Euthanize d mice | Aver age Dissected Tumor Weight (g) | Aver age Weight Before Dissection (g) | Aver age Tumor Volume at Endpoint (mm$^3$ ) | Experime ntal Endpoint TGI (%) |
|---|---|---|---|---|---|---|---|
| Group A (Control) | 8 | 8 | 0 | 0.92 | 21.36 | 961.7 5 | 0 |
| Group B (Gemcitabin e) | 8 | 6 | 2 | 0.68 | 21.13 | 605.4 1 | 40.0 |
| Group D (MNH04863 + Gemcitabine ) | 8 | 8 | 0 | 0.53 | 21.14 | 523.1 1 | 49.5 |

FIG. 16 shows the statistical analysis results of tumor volume curve during the experimental period that Group D (*Christensenella sp.* MNH04863 & gemcitabine) was significantly lower than Group A (negative control).

FIG. 17 shows the statistical analysis results of tumor volume at the endpoint that Group D (*Christensenella sp.* MNH04863 & gemcitabine) was significantly lower than that of Group A, and clearly lower than that of Group B.

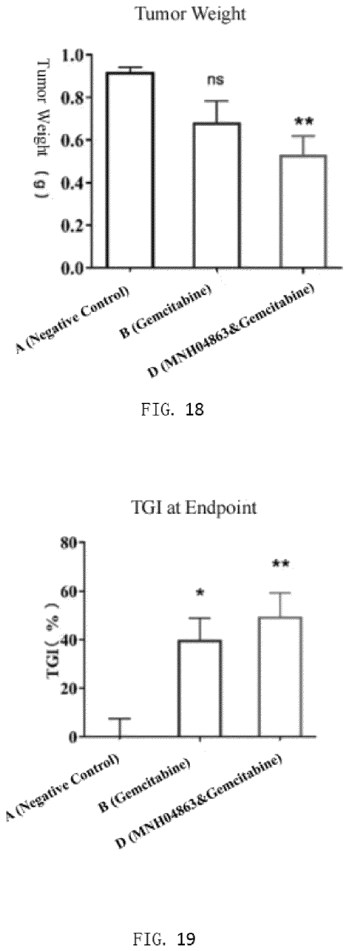

FIG. 18 shows the statistical analysis results of tumor weight at the endpoint that Group D (*Christensenella sp.* MNH04863 & gemcitabine) was significantly lower than that of Group A, and clearly lower than that of Group B.

FIG. 19 shows that the statistical analysis results of tumor growth inhibition rate (TGI) at the endpoint that Group D (*Christensenella sp.* MNH04863 & gemcitabine) was significantly higher than that of Group A, and clearly higher than that of Group B.

Example 12. Verification Experiment of the Therapeutic Effects of *Christensenella sp.* MNH04863 in Combination with Anti-PD-1 Antibody on Tumors of Pancreatic Cancer:

I. Experiment Methods:

To verify the therapeutic effects of *Christensenella sp.* MNH04863 in combination with anti-PD-1 antibody on tumor treatment, a mouse homologous tumor model was used to conduct experiments inhibiting pancreatic cancer growth. This experimental protocol has passed ethical review by the Laboratory Animal Management and Use Committee of Moon Biotech.

Test strain: The *Christensenella sp.* MNH04863 strain from a glycerol stock was thawed at 37°C and then inoculated onto anaerobic blood agar plates under anaerobic conditions for activation. The activated strain was subsequently inoculated into MM01 liquid medium for anaerobic cultivation to obtain sufficient viable bacteria. The cultured bacterial solution was then centrifuged and concentrated, and the bacterial pellet was resuspended in solvent to obtain the subjects with the purity and the number of viable bacteria ($1\times10^9$ CFU/mL~$2\times10^{10}$CFU/mL) that satisfy the requirements of the animal experiments.

Anti-PD-1 Antibody: Anti-PD-1 antibody (RMP1-14) was prepared as a 2 mg/mL solution in saline. Source: BioXcell.

Tumor Cells: KPC mouse pancreatic cancer cells; Source: Fubio (Suzhou) Biomedical Technology Co., Ltd., Catalog No.: FNC0071.

Experimental Animals: The experimental mice were C57BL/6J mice, aged 7-8 weeks, and purchased from Guangdong GemPharmatech Co., Ltd.

Animal Experiment: Under normal feeding conditions, after the quarantine, subcutaneous injection of KPC mouse pancreatic cancer cells was performed to form an ectopic homologous tumor model. The inoculation volume was $3\times10^6$ cells/mouse, 0.1~0.3 mL/mouse. When the average tumor volume reached 60-100 mm$^3$, animals were randomly divided into 10 groups based on tumor volume:

Group A (Negative Control);
Group B (Positive Control: anti-PD-1 antibody);
Group D (*Christensenella sp.* MNH04863 & anti-PD-1 antibody); and
Groups C and E-J are other test substances.

Oral gavage administration began on the day of grouping. Group A was administered with solvent and saline,

Group B was administered with solvent and anti-PD-1 antibody, and Group D was administered with *Christensenella sp.* MNH04863 and anti-PD-1 antibody. Solvent and *Christensenella sp.* MNH04863 were administered via oral gavage at a volume of 0.1~0.3 mL/mouse. When the average tumor volume in the negative control group A reached 120mm$^3$~150mm$^3$ or 7 days after gavage administration, anti-PD-1 antibody and saline were administered via intraperitoneal injection at a dose of 5~15mg/kg every 3 days for a total of 5 doses. Body weights of animals were recorded upon receipt of the animals and at the end of the quarantine. After tumor inoculation, body weights were measured twice weekly, including on days when intraperitoneal injections were administered. The day of tumor cell inoculation was designated as D1. From D5 until the grouping, tumor diameters were measured once daily; after grouping, tumor diameters were measured every 3 days. When the average tumor volume reached $\geq 600$ mm$^3$, tumor diameters were measured once daily, and tumor growth was recorded.

The experiment could be terminated if the average tumor volume in any group reached $\geq 600$ mm$^3$, and the tumor volume in any of Groups C to J was significantly smaller than that in Groups A and B, or the average tumor growth inhibition rate (TGI) in that group was $\geq 20\%$ higher than that of Group B. Alternatively, the experiment could be terminated if the average tumor volume in any group exceeded 2000 mm$^3$. General clinical observations, body weight monitoring, and tumor volume (mm$^3$) measurements were conducted throughout the experiment. At the experimental endpoint, all surviving mice were dissected for tumor tissue collection, and tumor weight was measured and tumor volume was calculated. Finally, statistical analyses and comparisons were performed on the tumor volume curve changes, endpoint tumor volume, endpoint tumor weight, and endpoint tumor growth inhibition rate (TGI) *etc.*

II. Experimental results of *Christensenella sp.* MNH04863 in combination with Anti-PD-1 antibody on tumor treatment of pancreatic cancer:

At the experimental endpoint (D33), the surviving mice were dissected and their tumor tissues weighed. The results are summarized in Table 16.

Table 16: Summary statistics of overall mouse conditions at experimental endpoint:

| Group | Number of Mice Enrolled | Number of mice at Experimental endpoint | Number of Euthanized mice | Average Weight Before Dissection (g) | Average Tumor Volume at Endpoint (mm$^3$) | Experimental Endpoint TGI (%) |
|---|---|---|---|---|---|---|
| Group A (Negative Control) | 8 | 8 | 0 | 21.26 | 958.42 | 0 |
| Group B (Positive control: anti-PD-1 antibody) | 8 | 8 | 0 | 22.53 | 871.39 | 9.78 |
| Group F *(Christensenella sp.* MNH04863 & anti-PD-1 antibody) | 8 | 8 | 0 | 21.38 | 782.09 | 19.83 |

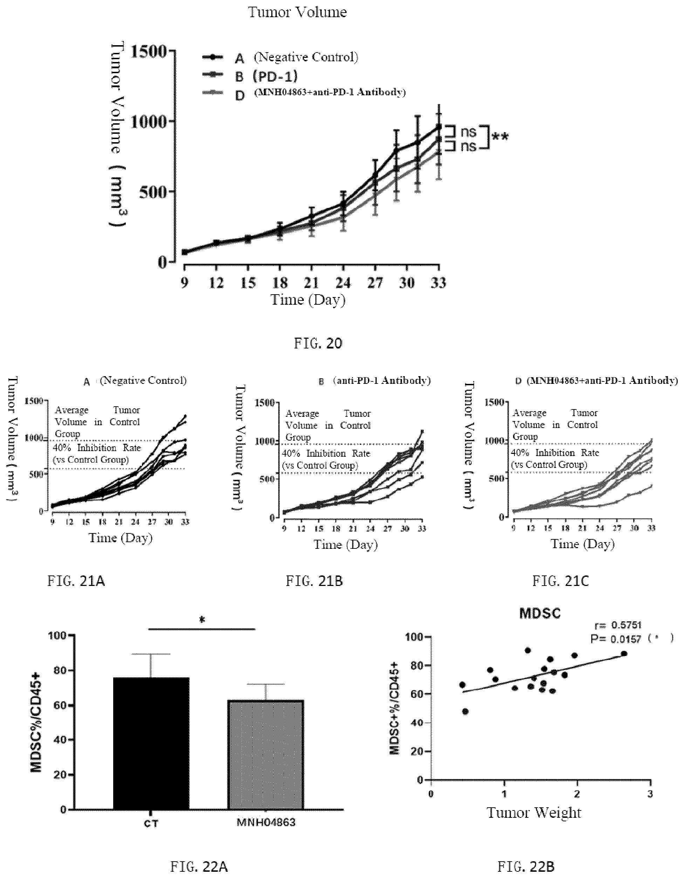

FIG. 20 shows a graph of the tumor volume curves of mice in this example. As shown in FIG. 20, during the experimental period, the statistical analysis results of tumor volume curve in Group D *(Christensenella sp.* MNH04863 & anti-PD-1 antibody) were significantly lower than that in Group A (negative control).

FIGs. 21A to 21C show the graphs of the response rates of mice at the experimental endpoint in this example. As shown in FIGs. 21A to 21C, at the experimental endpoint, the response rate was 0.0% in Group A (negative control), 12.5% in Group B (positive control), and 12.5% in Group D *(Christensenella sp.* MNH04863 & anti-PD-1 antibody).

Pancreatic cancer is recognized as a clinically refractory tumor type. As shown in Table 12, the anti-PD-1 antibody had very limited effects on reducing tumor volume and achieving tumor inhibition rate. In contrast, the combination treatment with *Christensenella sp.* MNH04863 and anti-PD-1 antibody effectively increased the tumor growth inhibition rate and significantly reduced tumor volume.

Example 13. Immune Analysis of *Christensenella sp.* MNH04863 in a Liver Cancer Model

Tumor tissues of the tumor-bearing mice from Example 8 were prepared to single-cell suspensions, labeled with fluorescently tagged specific molecules, and analyzed by flow cytometry to detect the proportion of cells expressing these molecules. The distribution of immune cells can reflect the immune state of mice in certain extent. The experimental steps are as follows:

Tumor tissues were harvested, rinsed with PBS, minced, and then homogenized. The homogenized tumor tissue was placed in a 5 mL collagenase IV digestion solution (1 mg/mL of collagenase IV, 0.1 mg/mL of DNase, 10% of FBS) at 37°C for 30 minutes. The digested tissue suspension was filtered through a 70 $\mu$m filter to produce a single-cell suspension of mouse tumors, which was then analyzed using a flow cytometer to count the total number of cells in the tissue. For cell surface staining, the single-cell suspension was stained with dyes (corresponding antibody combinations) Live/Fc and MDSC-Surface. Finally, the cells were resuspended in PBS for flow cytometric analysis.

Flow cytometry data were processed using CyExpert software, and statistical analyses were performed using GraphPad Prism V9. Statistical significance was determined using unpaired t-tests: ns, not significant; *p<0.05, **p<0.01, and ***p<0.001. Correlation analysis was conducted using the linear regression function, where Pearson r indicates correlation, and P value represents statistical significance.

As shown in FIGs. 22A and 22B, compared to the control group, treatment with *Christensenella sp.* MNH04863 significantly reduced the proportion of MDSC cells in liver cancer. Further efficacy correlation analysis in FIG. 22B shows that the effect of *Christensenella sp.* MNH04863 on MDSC proportions is positively correlated with tumor weight, indicating that changes in MDSC levels are associated with therapeutic efficacy.

These results suggest that in a liver cancer model, *Christensenella sp.* MNH04863 can enhance anti-tumor effects by reducing the proportion of MDSCs in the tumor microenvironment.

Example 14. Immune Analysis of *Christensenella sp.* MNH04863 in a Lung Cancer Model

Tumor tissues of the tumor-bearing mice from Example 6 were prepared to single-cell suspensions, labeled with fluorescently tagged specific molecules, and analyzed by flow cytometry to detect the proportion of cells expressing these molecules. The distribution of immune cells can reflect the immune status of mice in certain extent. The experimental steps are as follows:

Tumor tissues were harvested, rinsed with PBS, minced, and then homogenized. The homogenized tumor tissue was placed in a 5 mL collagenase IV digestion solution (1 mg/mL of collagenase IV, 0.1 mg/mL of DNase, 10% of FBS) at 37°C for 30 minutes. The digested tissue suspension was filtered through a 70 $\mu$m filter to produce a single-cell suspension of mouse tumors, which was then analyzed using a flow cytometer to count the total number of cells in the tissue. For CD4 and CD8 T cell analysis, the single-cell suspension was stained with dyes (corresponding antibody combinations) Live/Fc and T cell-Surface. Finally, the cells were resuspended in PBS and subjected to flow cytometric analysis. For Treg analysis, the suspension was sequentially stained with fluorescent dyes (corresponding antibody combinations) Live/Fc and Treg-Surface. Subsequently, according to the manufacturer's instructions of the detection kit, the cells were treated with fixed liquid and permeabilized liquid, followed by intranuclear staining using Treg-Foxp3 staining reagent. After staining, the cells were resuspended in PBS for flow cytometric analysis.

Flow cytometry data were processed using CyExpert software, and statistical analyses were performed using GraphPad Prism V9. Statistical significance was determined using unpaired t-tests: ns, not significant; *p<0.05, **p<0.01, and ***p<0.001.

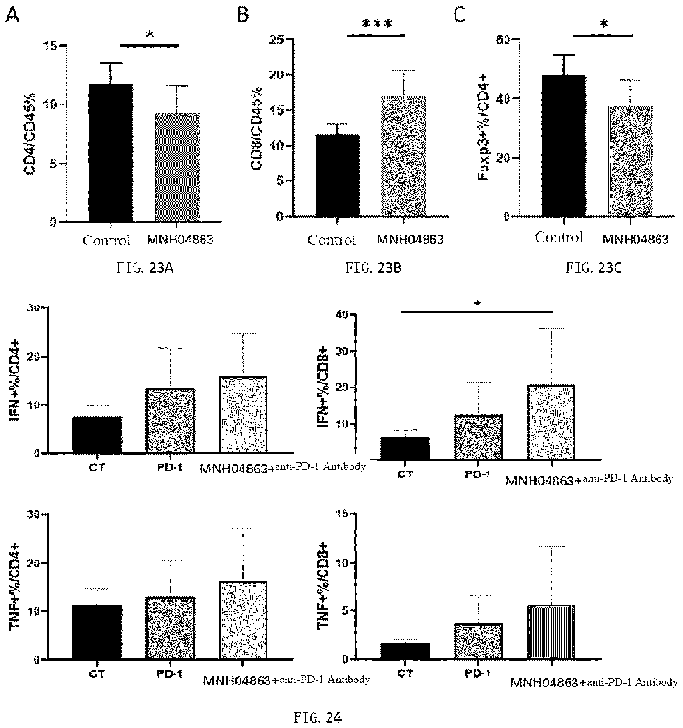

The results show that compared to the control group, the proportion of CD4+ T cells was significantly reduced under the effect of *Christensenella sp.* MNH04863 (FIG. 23A), whereas CD8+ T cells were significantly increased (FIG. 23B). Additionally, the proportion of Foxp3+ CD4+ T cells was significantly reduced under the influence of MNH04863 (FIG. 23C).

These results indicate that in a lung cancer model, *Christensenella sp.* MNH04863 can increase the proportion of tumor cytotoxic CD8+ T cells while reducing the proportion of immunosuppressive Foxp3+ CD4+ T cells, thereby enhancing anti-tumor effects.

Example 15. Immune Analysis of *Christensenella sp.* MNH04863 in Combination with Anti-PD-1 Antibody in a Lung Cancer Treatment Model

Tumor tissues of the tumor-bearing mice from Example 7 were prepared to single-cell suspensions, labeled with fluorescently tagged specific molecules, and analyzed by flow cytometry to detect the proportion of cells expressing these molecules. The distribution of immune cells can reflect the immune status of the mice in certain extent. The experimental steps are as follows:

Tumor tissues were harvested, rinsed with PBS, minced, and then homogenized. The homogenized tumor tissue was placed in a 5 mL collagenase IV digestion solution (1 mg/mL of collagenase IV, 0.1 mg/mL of DNase, 10% of FBS) at 37°C for 30 minutes. The digested tissue suspension was filtered through a 70 $\mu$m filter to produce a single-cell suspension of mouse tumors, which was then analyzed using a flow cytometer to count the total number of cells in the tissue. The mouse tumor single-cell suspension was stimulated with a cytokine stimulation solution containing PMA, Ionomycin, and BFA for 4 h. Subsequently, fluorescent dyes (corresponding antibody combinations), including Live/Fc and T-Surface, were added for cell surface staining. Following this, according to the manufacturer's instructions of the detection kit, the cells were treated with fixed liquid and permeabilized liquid, and then stained for the intracellular cytokines IFN-y and TNF-$\alpha$. After staining, the cells were resuspended in PBS and analyzed using a flow cytometer.

Flow cytometry data were processed using CyExpert software, and statistical analyses were performed using GraphPad Prism V9. Statistical significance was determined by one-way ANOVA with Dunnett's Multiple Comparison test: ns, not significant, *p<0.05, **p<0.01, and ***p<0.001.

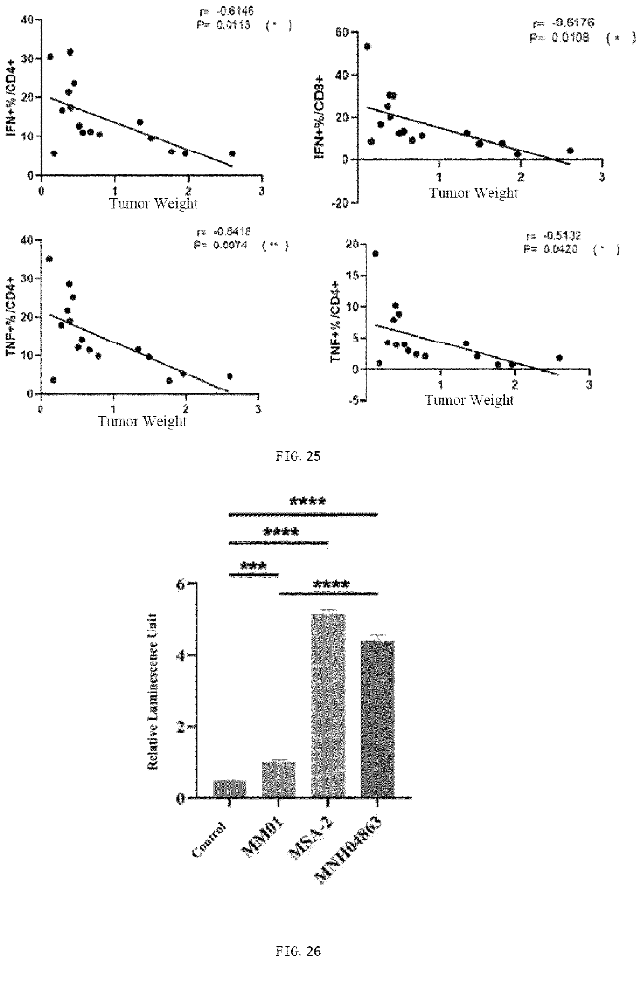

As shown in FIG. 24, compared to the control group, the combination of *Christensenella sp.* MNH04863 and anti-PD-1 antibody showed an increasing trend in the proportions of IFN-$\gamma^+$CD4$^+$, IFN-$\gamma^+$CD8$^+$, TNF-$\alpha^+$CD4$^+$, and TNF-$\alpha^+$CD8$^+$ T cells within the tumor tissue, with a statistically significant increase observed in IFN-$\gamma^+$CD8$^+$ T cells. Furthermore, pharmacodynamic correlation analysis was conducted to evaluate the relationship between these cell proportions and tumor weight. As shown in FIG. 25, when comparing the *Christensenella sp.* MNH04863 & anti-PD-1 antibody group with the anti-PD-1 antibody group alone, the proportions of CD4$^+$IFN-$\gamma^+$, CD8$^+$IFN-$\gamma^+$, CD4$^+$TNF-$\alpha^+$, and CD8$^+$TNF-$\alpha^+$ cells were significantly negatively correlated with tumor weight, consistent with the overall tumor treatment efficacy. In Example 7, the tumor weight of mice treated with *Christensenella sp.* MNH04863 in combination with anti-PD-1 antibody was significantly reduced compared to the control group. Combined with the results shown in FIG. 25, it can be confirmed that the combination of *Christensenella sp.* MNH04863 with anti-PD-1 antibody significantly increased the proportions of CD4$^+$IFN-$\gamma^+$, CD8$^+$IFN-$\gamma^+$, CD4$^+$TNF-$\alpha^+$, and CD8$^+$TNF-$\alpha^+$ cells. These results indicate that *Christensenella sp.* MNH04863 activates T cells and enhances the anti-tumor effect of anti-PD-1 antibodies.

Example 16. Effect of *Christensenella sp.* MNH04863 on the Expression of IFN$\beta$

Experiment Methods:

Interferon (IFN) receptor proteins are a class of cytokines secreted by host cells that regulate immune responses. Viruses, bacterial endotoxins, double-stranded RNA of artificial synthesis and the like can stimulate the production of interferons. Macrophages, lymphocytes, somatic cells, and others in the human body can all produce interferons. Among these, IFN$\beta$ belongs to Type I interferons, which can promote the activity of NK cells, macrophages, and T lymphocytes, thereby exerting antiviral, antitumor, and immunomodulatory effects. To verify whether *Christensenella sp.* MNH04863 can promote IFN$\beta$ expression, this study utilized THP-1 cells carrying an IFN$\beta$ gene promoter reporter (a proprietary cell line developed by Moon Biotechnology) to evaluate the effect of MNH04863 on the transcriptional activity of IFN$\beta$.

The construction steps for THP-1 cells carrying the IFN$\beta$ gene promoter reporter comprises: inserting the reporter gene into a vector, infecting cells with the vector, and screening to obtain a cell line that expresses the reporter gene (for more details, see the document: Huashan Du, Tianmin Xu, Manhua Cui "cGAS-STING signaling in cancer immunity and immunotherapy" Biomedicine & Pharmacotherapy 133 (2021) 110972; Jiang et al. "cGAS-STING, an important pathway in cancer immunotherapy" Journal of Hematology & Oncology (2020) 13:81; Khiem C. Lam et al. "Microbiota triggers STING-type I IFN-dependent monocyte reprogramming of the tumor microenvironment" Cell 184, 5338-5356).

Preparation of MNH04863 Culture Supernatant: Strain MNH04863 was inoculated into MM01 liquid medium and anaerobically cultured at 37°C for 48 hours. The culture supernatant was centrifuged to remove bacterial pellet, filtered through a 0.22 $\mu$m filter, aliquoted, and stored at -80°C for later use.

Control Group: DMEM complete medium containing 10% volume of MM04863 bacterial medium (10% FBS).

Positive Control: MSA-2 (40 $\mu$M), sourced from TargetMol Cat No. T8798 (Target Molecule Corp.). MSA-2 is an orally available non-nucleotide STING agonist that binds to STING as a non-covalent dimer with nanomolar affinity, promoting IFN$\beta$ expression.

MNH04863 Group: Comprising 10% volume of MNH04863 bacterial culture supernatant.

THP-1 cells carrying the IFN$\beta$ gene promoter reporter were inoculated into 96-well plates at $1 \times 10^5$ cells per well. Cells were treated according to the specified groups. After continuing culture for 24 hours, cells were centrifuged at 300 g for 5 minutes, and the culture supernatant was removed. Then, 50 $\mu$L of $1 \times$ luciferase assay reagent was added, reacted for 1 min, and luminescence values were measured using a plate reader. Relative luminescence units (RLU) were normalized against the control group to evaluate the impact of MNH04863 on IFN$\beta$ transcriptional activity.

Experimental Results are shown in FIG. 26, MNH04863 significantly promotes IFN$\beta$ transcriptional activity, with efficacy comparable to that of the positive drug (STING agonist) in promoting Type I interferon IFN$\beta$. Type I interferon IFN$\beta$ has been confirmed to achieve antitumor effects through immune modulation and may have potential functions against viral tumors. Therefore, these results indicate that MNH04863 achieves antitumor effects through immune modulation and may have potential functions against viral tumors.

The medicament prepared from *Christensenella sp.* provided by the present invention can significantly promote IFN$\beta$ transcriptional activity, with therapeutic efficacy comparable to that of the positive drug (STING agonist) in enhancing Type I interferon IFN$\beta$. Type I interferon IFN$\beta$ has been demonstrated to reconstruct the synergy between innate and adaptive immunity within the tumor microenvironment, making it effective for the treatment of refractory, and drug-resistant cancers (see "Targeting the Tumor Microenvironment with Interferon-β Bridges Innate and Adaptive Immune Responses," Yang X1, Cancer Cell, 2014, doi: 10.1016/j.ccr.2013.12.004). Therefore, these results indicate that MNH04863 or the medicament prepared from *Christensenella sp.* provided by the present invention can achieve antitumor effects through immune modulation and may also have potential therapeutic functions against virus-associated tumors.

Finally, it should be noted that the above examples are only used to illustrate the technical solutions of the present invention, not to limit them; although the present invention has been described in detail with reference to the above examples, those skilled in the art will readily understand that modifications may still be made to the technical solutions described in the examples, or some or all of the technical features may be substituted with equivalent alternatives. Such modifications or substitutions do not cause the essence of the corresponding technical solutions to depart from the scope of the technical solutions disclosed in the embodiments of the present invention.

## Claims

1. A composition for preventing or treating a tumor, comprising a *Christensenella sp.,* or a metabolite, a fermentation culture, or a fermentation culture supernatant thereof, or any combination thereof;
   wherein the 16S rDNA sequence of the *Christensenella sp.* has at least 99%, 99.5%, 99.6%, 99.7%, 99.8%, 99.9%, 99.99%, or 100% identity to a sequence as shown in SEQ ID NO:1.

2. The composition for preventing or treating a tumor according to claim 1, wherein the *Christensenella sp.* comprises one or more bacteria selected from the following: Gram-negative *Christensenella intestinihominis, Christensenella massiliensis, Christensenella minuta, Christensenella timonensis,* or any combination thereof.

3. The composition for preventing or treating a tumor according to claim 1 or 2, wherein the *Christensenella sp.* is a Gram-negative bacterium; preferably, a Gram-negative *Christensenella intestinihominis;* more preferably, the 16S rRNA of the *Christensenella sp.* has at least 99%, 99.5%, 99.6%, 99.7%, 99.8%, 99.9%, 99.99%, or 100% identity with that of Gram-negative *Christensenella intestinihominis;* even more preferably, the *Christensenella sp.* comprises a strain with a deposit number of GDMCC No: 61117.

4. The composition for preventing or treating a tumor according to any one of the preceding claims, comprising an effective amount of a *Christensenella sp.,* or a metabolite, a fermentation culture, or a fermentation culture supernatant thereof, or any combination thereof; optionally, the composition further comprises a pharmaceutically acceptable or food-acceptable carrier or excipient, and/or probiotics.

5. A pharmaceutical composition for preventing or treating a tumor, comprising an effective amount of: (1) the composition according to any one of claims 1 to 4, and (2) at least one other agent selected from a chemotherapeutic drug, a radiotherapeutic drug, a targeted drug, a photosensitizer, a photothermal agent, an immunotherapeutic agent, a drug for releasing from immunosuppression or any combination thereof.

6. The pharmaceutical composition according to claim 5, wherein the chemotherapeutic drug is selected from at least one of: nitrogen mustard, cyclophosphamide, ifosfamide, melphalan, chlorambucil, hexamethylmelamine, thiotepa, busulfan, carmustine, lomustine, chlorozotocin, streptozotocin, dacarbazine, paclitaxel, methotrexate, 6-mercaptopurine, 6-thioguanine, pentostatin, vinblastine, vincristine, etoposide, teniposide, actinomycin D, daunorubicin, doxorubicin, mitomycin, epirubicin, bleomycin, plicamycin, mitoxantrone, L-asparaginase, interferon-a, procarbazine, camptothecin, paclitaxel, mitotane, 5-fluorouracil, cisplatin, carboplatin, hydroxyurea, gemcitabine, capecitabine, cytarabine, or azauracil;

   preferably, the chemotherapeutic drug is selected from gemcitabine; and/or
   the radiotherapeutic drug is selected from a drug used in external radiation therapy, internal radiation therapy, radioimmunotherapy, or intraoperative radiation therapy (IORT); and/or
   the photosensitizer is selected from boron dipyrromethene, chlorin, and/or rose bengal; and/or
   the photothermal agent is selected from indocyanine green, new indocyanine green, and/or gold nanorods; and/or
   the immunotherapeutic agent is selected from an immune checkpoint inhibitor, a co-stimulatory molecule inhibitor, a dendritic cell, a CAR-T cell, a cytokine, and/or an adoptive T cell; preferably, the immune checkpoint inhibitor is selected from an anti-PD-1 antibody, an anti-CTLA-4 antibody, an anti-PD-L1 antibody, and/or a PD-L1 inhibitor; preferably, the immune checkpoint inhibitor is selected from durvalumab, atezolizumab, avelumab, pembrolizumab, nivolumab, ipilimumab, or any combination thereof; and/or
preferably, the immunotherapeutic agent is selected from a monoclonal antibody, an immune checkpoint inhibitor, an immune cell, an oncolytic viruse, a tumor vaccine, or any combination thereof;
preferably, the immune checkpoint inhibitor is an inhibitor targeting T-cell molecules and/or their respective ligands; and/or
the drug for releasing from immunosuppression is an inhibitor that reduces or suppresses a proportion of MDSC.

7. The pharmaceutical composition according to claim 5 or 6, wherein the composition comprises: (1) *Christensenella sp.* and an anti-PD-1 antibody; or (2) *Christensenella sp.* and gemcitabine.

8. The pharmaceutical composition according to any one of claims 5 to 7, wherein the composition further comprises at least one probiotic selected from: *Bifidobacterium spp., Lactobacillus spp., Lactococcus spp., Lacticaseibacillus spp., Pediococcus acidilactici, Pediococcus pentosaceus,* and/or *Saccharomyces boulardii;* preferably, the probiotic is selected from: *Bifidobacterium adolescentis, Bifidobacterium animalis* subsp. *animalis, Bifidobacterium animalis* subsp. *lactis, Bifidobacterium bifidum, Bifidobacterium breve, Bifidobacterium longum* subsp. *longum, Bifidobacterium longum* subsp. *infantis, Lactobacillus acidophilus, Lactobacillus crispatus, Lactobacillus delbrueckii* subsp. *Bulgaricus, Lactobacillus delbrueckii* subsp. *Lactis, Lactobacillus gasseri, Lactobacillus helveticus, Lactobacillus johnsonii, Lactobacillus kefiranofaciens* subsp. *Kefiranofaciens, Lacticaseibacillus casei, Lacticaseibacillus paracasei, Lacticaseibacillus rhamnosus, Limosilactobacillus fermentum, Limosilactobacillus reuteri, Lactiplantibacillus plantarum, Ligilactobacillus salivarius, Latilactobacillus curvatus, Latilactobacillus sakei, Streptococcus salivarius* subsp. *thermophilus, Lactococcus lactis* subsp. *lactis, Lactococcus lactis* subsp. *lactis biovardiacetylactis, Lactococcus cremoris, Propionibacterium freudenreichii* subsp. *shermanii, Acidipropionibacterium acidipropionici, Leuconostoc mesenteroides* subsp. *mesenteroides, Pediococcus acidilactici, Pediococcus pentosaceus, Weizmannia coagulans, Mammaliicoccus vitulinus, Staphylococcus xylosus, Staphylococcus carnosus,* and *Kluyveromyces marxianus.*

9. The composition for preventing or treating a tumor according to any one of claims 1 to 4, or the pharmaceutical composition according to any one of claims 5 to 8, wherein the composition for preventing or treating a tumor or the pharmaceutical composition has one or more of the following characteristics (1) to (7):

   (1) being an infant-applicable dosage form, a child-applicable dosage form, or an adult-applicable dosage form;
   (2) being a dosage form for gastrointestinal administration or a dosage form for parenteral administration;
   (3) being an oral preparation or injectable preparation;
   (4) the *Christensenella sp.* being at least partially capable of proliferating in the intestinal tract of a subject;
   (5) the mass percentage of the *Christensenella sp.* or the metabolite, fermentation culture or fermentation culture supernatant thereof in the pharmaceutical composition being of 1% to 80%;
   (6) the *Christensenella sp.* being in a form selected from: live bacteria, attenuated bacteria, killed bacteria, lyophilized bacteria or irradiated bacteria; and
   (7) each gram of the composition for preventing or treating a tumor or the pharmaceutical composition comprising $1\times10^3$ to $1\times10^{13}$ colony forming units (CFU) of the *Christensenella sp.*

10. The composition for preventing or treating a tumor according to any one of claims 1 to 4, or the pharmaceutical composition according to any one of claims 5 to 8, wherein the composition for preventing or treating a tumor or the pharmaceutical composition is in the form of a liquid, foam, cream, spray, powder, capsule, or gel; preferably, it is in the form of a spray-dried powder, lyophilized powder, microcapsule, soft capsule, or enteric-coated capsule; more preferably, the composition for preventing or treating a tumor or the pharmaceutical composition is in the form selected from a live bacterial suspension, electrostatic spray-dried powder, soft capsule, or enteric-coated capsule.

11. The composition for preventing or treating a tumor according to any one of claims 1 to 4, and 9 to 10, wherein the composition for preventing or treating a tumor is a pharmaceutical composition, health product, or food, and optionally comprises a pharmaceutically acceptable or food-acceptable carrier or excipient.

12. Use of the composition according to any one of claims 1 to 11 in the preparation of a medicament for preventing or treating a tumor or inflammatory disease.

13. The use according to claim 12, wherein the tumor is selected from a solid tumor, a soft tissue tumor, a hematopoietic tumor, a glandular tumor, or a metastatic tumor; or the tumor is a tumor induced by a virus or a tumor induced by bacteria; or the tumor has elevated myeloid-derived suppressor cells (MDSCs);

preferably, the tumor is a cold tumor;

preferably, the tumor is a clinically refractory tumor or drug-resistant tumor; more preferably, the clinically refractory tumor includes a tumor with low response to PD-1 drug; the drug-resistant tumor includes PD-1 drug-resistant tumor, chemical drug-resistant tumor, chemotherapeutic drug-resistant tumor, and/or immunotherapeutic agent-resistant tumor; the clinically refractory tumor or drug-resistant tumor includes at least one of lung cancer, liver cancer, or pancreatic cancer;

more preferably, the tumor includes an immunotherapeutic agent-resistant tumor, such as a tumor resistant to anti-PD-1 antibody, anti-CTLA-4 antibody, anti-PD-L1 antibody, and/or PD-L1 inhibitor;

preferably, the tumor is selected from one or more of the following: liver cancer, colon cancer, rectal cancer, colorectal cancer, lung cancer, breast cancer, cervical cancer, ovarian cancer, pancreatic cancer, cholangiocarcinoma, kidney cancer, glioma, liposarcoma, chondrosarcoma, fibrosarcoma, melanoma, cervical cancer, neuroblastoma, lymphoma, leukemia, gastric cancer, hematologic malignancies, prostate cancer, small-cell lung cancer, squamous cell carcinoma, head and neck neoplasm, bladder cancer, nasopharyngeal carcinoma, multiple myeloma, HPV infection-induced tumor, HBV infection-induced tumor, HCV infection-induced tumor, HIV infection-induced tumor, *Helicobacter pylori* infection-induced tumor, EBV infection-induced tumor, or *Fusobacterium nucleatum* infection-induced tumor; even more preferably, the tumor is selected from one or more of the following: melanoma, glioma, head and neck neoplasm, lung cancer, colorectal cancer, bladder cancer, nasopharyngeal carcinoma, liver cancer, kidney cancer, pancreatic cancer, lymphoma (preferably cutaneous T-cell lymphoma or peripheral T-cell lymphoma), ovarian cancer, fibrosarcoma, multiple myeloma; and/or

the inflammatory disease is selected from rheumatic disease, connective tissue disease, systemic vasculitis, dermatological inflammatory disease, sepsis/systemic inflammatory response syndrome, acute respiratory distress syndrome, gastritis, pneumonia or hepatitis, preferably the inflammatory disease is selected from rheumatic arthritis, systemic sclerosis, systemic lupus erythematosus, sicca syndrome, Reiter's syndrome, systemic juvenile idiopathic arthritis, spondyloarthropathies, giant cell arteritis, polymyalgia rheumatica, aortoarteritis, polyarteritis nodosa, Kawasaki disease, ANCA-associated vasculitis, immune-complex-mediated vasculitis, Behcet's syndrome, relapsing polychondritis, sarcoidosis, psoriasis, psoriatic arthritis, eczema, chronic urticaria, neutrophilic dermatosis, acute or chronic gastritis, alopecia areata, asthma, bronchitis, or alcoholic or autoimmune hepatitis.

14. The use according to claim 12 or 13, wherein the medicament prevents and/or treats a tumor through at least one of the following pathways: (a) inhibiting tumor volume growth; (b) inhibiting tumor weight gain; (c) inhibiting tumor cell proliferation; (d) improving tumor treatment response rate; (e) improving the therapeutic efficacy of an immunosuppression drug, such as improving the therapeutic efficacy of a PD-1 drug; (f) inhibiting metastasis of tumor cells; (g) reducing PD-1 resistance; (h) promoting IFNβ transcriptional activity; (i) inhibiting a tumor via at least one of short-chain fatty acids or short-chain fatty acid salts including acetic acid or acetate, propionic acid or propionate, butyric acid or butyrate, and valeric acid or valerate in SCFAs; (j) inhibiting a tumor by modulating immune system in a subject to exert an immunomodulatory effect; (k) inhibiting a tumor by reducing the proportion of MDSCs in the tumor microenvironment; and (1) promoting or activating the subject's immune system (e.g., increasing CD4$^+$ or CD8$^+$ T-cell levels) to kill tumor or inhibit tumor growth.

15. The use according to any one of claims 12 to 14, wherein the medicament prevents or treats a tumor by enhancing the efficacy of one or more tumor therapies;

preferably, the medicament prevents or treats a tumor by immunomodulating macrophages, and/or primary PBMCs, and/or monocyte-derived dendritic cells; and/or
the medicament prevents or treats a tumor by promoting IFNβ transcriptional activity; and/or
the tumor has elevated MDSCs, preferably the tumor is a cold tumor; more preferably, the tumor is a drug-resistant tumor, preferably the tumor is an immunotherapeutic agent-resistant tumor, further preferably, the tumor is a tumor resistant to anti-PD-1 antibody, anti-CTLA-4 antibody, anti-PD-L1 antibody, and/or PD-L1 inhibitor.

16. Use of the composition according to any one of claims 1 to 11 in the preparation of a medicament, wherein the medicament is used as an immunomodulator.

17. The use according to claim 16, wherein the medicament inhibits tumor growth by reducing a proportion of MDSCs in the tumor microenvironment or activates an immune system (e.g., increasing CD4$^+$ or CD8$^+$ T-cell levels) to kill tumor or inhibit tumor growth.

**18.** The use according to claim 16 or 17, wherein the tumor is selected from a solid tumor, a soft tissue tumor, a hematopoietic tumor, a glandular tumor, or a metastatic tumor; or the tumor is a tumor induced by a virus or a tumor induced by bacteria; or the tumor has elevated myeloid-derived suppressor cells (MDSCs);

preferably, the tumor is a cold tumor;
preferably, the tumor is a clinically refractory tumor or drug-resistant tumor; more preferably, the clinically refractory tumor includes a tumor with low response to PD-1 drug; the drug-resistant tumor includes PD-1 drug-resistant tumor, chemical drug-resistant tumor, chemotherapeutic drug-resistant tumor, and/or immunotherapeutic agent-resistant tumor; the clinically refractory tumor or drug-resistant tumor includes at least one of lung cancer, liver cancer, or pancreatic cancer;
more preferably, the tumor includes an immunotherapeutic agent-resistant tumor, such as a tumor resistant to anti-PD-1 antibody, anti-CTLA-4 antibody, anti-PD-L1 antibody, and/or PD-L1 inhibitor;
preferably, the tumor is selected from one or more of the following: liver cancer, colon cancer, rectal cancer, colorectal cancer, lung cancer, breast cancer, cervical cancer, ovarian cancer, pancreatic cancer, cholangiocarcinoma, kidney cancer, glioma, liposarcoma, chondrosarcoma, fibrosarcoma, melanoma, cervical cancer, neuroblastoma, lymphoma, leukemia, gastric cancer, hematologic malignancies, prostate cancer, small-cell lung cancer, squamous cell carcinoma, head and neck neoplasm, bladder cancer, nasopharyngeal carcinoma, multiple myeloma, HPV infection-induced tumor, HBV infection-induced tumor, HCV infection-induced tumor, HIV infection-induced tumor, *Helicobacter pylori* infection-induced tumor, EBV infection-induced tumor, or *Fusobacterium nucleatum* infection-induced tumor; even more preferably, the tumor is selected from one or more of the following: melanoma, glioma, head and neck neoplasm, lung cancer, colorectal cancer, bladder cancer, nasopharyngeal carcinoma, liver cancer, kidney cancer, pancreatic cancer, lymphoma (preferably cutaneous T-cell lymphoma or peripheral T-cell lymphoma), ovarian cancer, fibrosarcoma, multiple myeloma.

**19.** A method for treating a tumor, comprising administering to a subject *Christensenella sp.* or the composition according to any one of claims 1 to 11;
**characterized in that** *Christensenella sp.* in the composition satisfies at least one of A1) to A4):

A1) 16S rRNA of the *Christensenella sp.* has at least 99%, 99.5%, 99.6%, 99.7%, 99.8%, 99.9%, 99.99%, or 100% identity with the sequence as shown in SEQ ID NO: 1;
A2) the Christensenella sp. is selected from Gram-negative Christensenella intestinihominis, Christensenella massiliensis, Christensenella minuta, Christensenella timonensis, or any combination thereof;
preferably, the *Christensenella sp.* is Gram-negative *Christensenella intestinihominis;* more preferably, the *Christensenella sp.* comprises a strain with a deposit number of GDMCC No: 61117;
A3) the genome of the *Christensenella sp.* has an average nucleotide identity of 294%, ≥95%, ≥95.5%, ≥96%, ≥96.5%, ≥97%, ≥97.5%, ≥98%, ≥98.5%, ≥99%, ≥99.5%, ≥99.6%, ≥99.7%, ≥99.8%, ≥99.9%, ≥99.99%, or ≥100% with the genome of strain MNH04863 with a deposit number of GDMCC No: 61117;
an alignment score of the genome of *Christensenella sp.* with that of MNH04863 with a deposit number of GDMCC No: 61117 is ≥78%, ≥80%, ≥85%, ≥90%, ≥95%, ≥96%, ≥97%, ≥98%, or ≥99%; and
A4) 16S rRNA of the *Christensenella sp.* has at least 99%, 99.5%, 99.6%, 99.7%, 99.8%, 99.9%, 99.99%, or 100% identity with that of strain of GDMCC No: 61117.

FIG. 1

FIG. 2

## MNH04863 Bile Salt Tolerance

FIG. 3

FIG. 4

FIG. 5

FIG. 6

Tumor Volume

FIG. 7

Tumor Volume at Endpoint

FIG. 8

Tumor Weight

FIG. 9

Tumor Growth Inhibition at Endpoint

FIG. 10A

Tumor Growth Inhibition

GroupA(Negative Control)
GroupB(MNH04863)

FIG. 10B

FIG. 11A

FIG. 11B

FIG. 12

FIG. 13

FIG. 14

44

TGI at Endpoint

FIG. 15

Tumor Volume

FIG. 16

Tumor Volume at Endpoint

FIG. 17

Tumor Weight

FIG. 18

TGI at Endpoint

FIG. 19

FIG. 20

FIG. 21A

FIG. 21B

FIG. 21C

FIG. 22A

FIG. 22B

FIG. 23A    FIG. 23B    FIG. 23C

FIG. 24

FIG. 25

FIG. 26

## INTERNATIONAL SEARCH REPORT

| International application No. |
| --- |
| **PCT/CN2023/142542** |

| A. | CLASSIFICATION OF SUBJECT MATTER |
| --- | --- |

A61K35/74(2015.01)i;  C12N1/20(2006.01)i;  A23L33/135(2016.01)i;  A61P29/00(2006.01)i;  A61P35/00(2006.01)i

According to International Patent Classification (IPC) or to both national classification and IPC

| B. | FIELDS SEARCHED |
| --- | --- |

Minimum documentation searched (classification system followed by classification symbols)

IPC:A61K,C12N, A23L,A61P

Documentation searched other than minimum documentation to the extent that such documents are included in the fields searched

Electronic data base consulted during the international search (name of data base and, where practicable, search terms used)

CNTXT, WPABSC, ENTXTC, VEN, DWPI, WPABS, ENTXT, CJFD, 中国专利生物序列检索, China Patent Biological Sequence Search, Genbank, EMBL-EBI, ISI-Web of science, CNKI, 超星读秀, CHAOXING DUXIU, 百度学术, Baidu Scholar: 克里斯滕森, 克里斯坦森, 克里斯特氏, MNH, MNO, +863, +4863, GDMCC, +61117, 菌株, 代谢产物, 发酵培养液, 发酵培养液上清, 瘤, 癌, 炎症, 炎性疾病, 益生菌, 免疫调节, 免疫抑制, 免疫激活, 激活免疫, CD4, CD8, MDSC, 根据核苷酸序列SEQ ID No.: 1进行了序列检索, search according to nucleotide sequence SEQ ID No: 1, Christensenella, Christensenella intestinihominis, Christensenella massiliensis, Christensenella minuta, Christensenella timonensis, Strains, metabolites, fermentation broth, fermentation broth supernatant, Tumor, Cancer, Inflammation, inflammatory diseases, Probiotic, Immunomodulat+, Immunosuppress+, Immune activat+, Activat+ immunity

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 113631174 A (YSOPIA BIOSCIENCES; INSTITUT NATIONAL DE RECHERCHE POUR L'AGRICULTURE, L'ALIMENTATION ET L'ENVIRONNEMENT; ALMA MATER STUDIORUM - UNIVERSITA' DI BOLOGNA) 09 November 2021 (2021-11-09) claims 1-22, and description, paragraphs 3-10 and 44-127 | 1-6, 8-19 |
| Y | CN 113631174 A (YSOPIA BIOSCIENCES; INSTITUT NATIONAL DE RECHERCHE POUR L'AGRICULTURE, L'ALIMENTATION ET L'ENVIRONNEMENT; ALMA MATER STUDIORUM - UNIVERSITA' DI BOLOGNA) 09 November 2021 (2021-11-09) claims 1-22, and description, paragraphs 3-10 and 44-127 | 7 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 April 2024** | **15 April 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/142542** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 110582291 A (INSTITUT GUSTAVE ROUSSY; INSTITUT NATIONAL DE LA SANTE ET DE LA RECHERCHE MEDICALE (INSERM); UNIVERSITE PARIS SUD; INSTITUT NATIONAL DE LA RECHERCHE AGRONOMIQUE (INRA)) 17 December 2019 (2019-12-17)<br>claims 1-36 | 5-8, 12-19 |
| X | CN 113230387 A (MOON (GUANGZHOU) BIOTECH CO., LTD.) 10 August 2021 (2021-08-10)<br>claims 1-10, and description, paragraphs 6-30 and 46-88 | 1-4, 9-19 |
| Y | CN 113230387 A (MOON (GUANGZHOU) BIOTECH CO., LTD.) 10 August 2021 (2021-08-10)<br>claims 1-10, and description, paragraphs 6-30 and 46-88 | 5-8 |
| X | WO 2018045493 A1 (BGI SHENZHEN) 15 March 2018 (2018-03-15)<br>claims 1-9, and description, page 2, line 7 to page 4, line 20 | 1-4, 9-11 |
| Y | WO 2018045493 A1 (BGI SHENZHEN) 15 March 2018 (2018-03-15)<br>claims 1-9, and description, page 2, line 7 to page 4, line 20 | 5-8, 12-19 |
| X | US 2016287636 A1 (INT NUTRITION RES COMPANY) 06 October 2016 (2016-10-06)<br>claims 1-18 | 1-19 |
| X | ZOU, Yuanqiang et al. "Taxonomic Description and Genome Sequence of Christensenella intestinihominis sp. nov., a Novel Cholesterol-Lowering Bacterium Isolated From Human Gut" *Frontiers in Microbiology,* Vol. vol. 12, 22 February 2021 (2021-02-22),<br>page 1, abstract, and page 11, left-hand column, paragraph 2 | 1-4, 9-11 |

Form PCT/ISA/210 (second sheet) (July 2022)

## INTERNATIONAL SEARCH REPORT

| International application No. |
|---|
| **PCT/CN2023/142542** |

| **Box No. I** | **Nucleotide and/or amino acid sequence(s) (Continuation of item 1.c of the first sheet)** |
|---|---|

1.  With regard to any nucleotide and/or amino acid sequence disclosed in the international application, the international search was carried out on the basis of a sequence listing:

   a.  ☐ forming part of the international application as filed.

   b.  ☑ furnished subsequent to the international filing date for the purposes of international search (Rule 13*ter*.1(a)),

   ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/142542** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **19**
because they relate to subject matter not required to be searched by this Authority, namely:

Claim 19 sets forth "a method for treating tumors", which relates to a method for treating diseases, and falls within subject matter for which an international search is not required as defined in PCT Rule 39.1(iv). The examiner conducts a search on the basis of a reasonable expectation, i.e., the subject matter of claim 19 is amended to be "the use of Christensenella or the composition of any one of claims 1-11 in the preparation of a drug for treating tumors".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2023/142542** |

| Patent document cited in search report | | | Publication date (day/month/year) | Patent family member(s) | | | Publication date (day/month/year) |
|---|---|---|---|---|---|---|---|
| CN | 113631174 | A | 09 November 2021 | CA | 3132239 | A1 | 17 September 2020 |
| | | | | AU | 2020235358 | A1 | 07 October 2021 |
| | | | | SG | 11202109872 | SA | 28 October 2021 |
| | | | | KR | 20210141541 | A | 23 November 2021 |
| | | | | WO | 2020182916 | A1 | 17 September 2020 |
| | | | | FR | 3093642 | A1 | 18 September 2020 |
| | | | | FR | 3099054 | A1 | 29 January 2021 |
| | | | | IL | 286182 | A | 31 October 2021 |
| | | | | BR | 112021017950 | A2 | 16 November 2021 |
| | | | | EP | 3937958 | A1 | 19 January 2022 |
| | | | | MX | 2021010868 | A1 | 25 March 2022 |
| | | | | JP | 2022525058 | W | 11 May 2022 |
| | | | | US | 2023167401 | A1 | 01 June 2023 |
| CN | 110582291 | A | 17 December 2019 | EP | 3559257 | A1 | 30 October 2019 |
| | | | | US | 2023263843 | A1 | 24 August 2023 |
| | | | | IL | 267564 | A | 29 August 2019 |
| | | | | AU | 2017380257 | A1 | 04 July 2019 |
| | | | | CA | 3047029 | A1 | 28 June 2018 |
| | | | | WO | 2018115519 | A1 | 28 June 2018 |
| | | | | KR | 20190118151 | A | 17 October 2019 |
| | | | | KR | 102644935 | B1 | 07 March 2024 |
| | | | | US | 2021346438 | A1 | 11 November 2021 |
| | | | | US | 11684640 | B2 | 27 June 2023 |
| | | | | JP | 2020512294 | A | 23 April 2020 |
| | | | | JP | 7366744 | B2 | 23 October 2023 |
| | | | | JP | 2023133291 | A | 22 September 2023 |
| | | | | SG | 11201905246 | A1 | 30 July 2019 |
| CN | 113230387 | A | 10 August 2021 | CN | 117065006 | A | 17 November 2023 |
| WO | 2018045493 | A1 | 15 March 2018 | US | 2019282633 | A1 | 19 September 2019 |
| | | | | US | 10806759 | B2 | 20 October 2020 |
| | | | | JP | 2019517992 | A | 27 June 2019 |
| | | | | JP | 6782302 | B2 | 11 November 2020 |
| | | | | EP | 3511407 | A1 | 17 July 2019 |
| | | | | EP | 3511407 | A4 | 08 April 2020 |
| | | | | EP | 3511407 | B1 | 17 May 2023 |
| | | | | CN | 109072173 | A | 21 December 2018 |
| | | | | HK | 40000506 | A0 | 07 February 2020 |
| | | | | HK | 40000506 | A0 | 07 February 2020 |
| US | 2016287636 | A1 | 06 October 2016 | US | 9616092 | B2 | 11 April 2017 |
| | | | | DK | 3277295 | T3 | 26 July 2021 |
| | | | | EP | 3277295 | A1 | 07 February 2018 |
| | | | | EP | 3277295 | B1 | 28 April 2021 |
| | | | | ES | 2881377 | T3 | 29 November 2021 |
| | | | | PT | 3277295 | T | 22 July 2021 |
| | | | | US | 2017065637 | A1 | 09 March 2017 |
| | | | | US | 10064895 | B2 | 04 September 2018 |
| | | | | FR | 3034317 | A1 | 07 October 2016 |
| | | | | FR | 3034317 | B1 | 14 September 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113069475 A

- WO 2018045493 A1

**Non-patent literature cited in the description**

- **JIANJIAO NI.** Molecular Mechanisms and Clinical Significance of STING Pathway Regulation of Myeloid-Derived Suppressor Cells in Nasopharyngeal Carcinoma [D].. *Peking Union Medical College, Chinese Academy of Medical Sciences*, 26 December 2023
- **YANG X1**. Targeting the Tumor Microenvironment with Interferon-β Bridges Innate and Adaptive Immune Responses. *Cancer Cell*, 2014

- **HUASHAN DU** ; **TIANMIN XU** ; **MANHUA CUI**. cGAS-STING signaling in cancer immunity and immunotherapy. *Biomedicine & Pharmacotherapy*, 2021, vol. 133, 110972
- **JIANG et al.** cGAS-STING, an important pathway in cancer immunotherapy. *Journal of Hematology & Oncology*, 2020, vol. 13, 81
- **KHIEM C. LAM et al.** Microbiota triggers STING-type I IFN-dependent monocyte reprogramming of the tumor microenvironment. *Cell*, vol. 184, 5338-5356